# EUROPEAN PATENT APPLICATION

(11) **EP 4 763 284 A1**
(43) Date of publication of application: **24.06.2026**
(21) Application number: 25225019.6
(22) Date of filing: 18.12.2025
(51) Int. Cl.: A61P 35/00, C07D 471/04, A61K 31/437

(54) **TOLL-LIKE RECEPTOR AGONIST-BASED VACCINE ADJUVANT LIPID COMPOUND AND USE THEREOF**

(30) Priority: 19.12.2024 CN 202411874747
(71) Applicant: Hangzhou Tianlong Pharmaceutical Co., Ltd., Hangzhou, Zhejiang 310020 (CN)
(72) Inventor: ZHANG, Honglei, Hangzhou, 310020 (CN); SONG, Gengshen, Hangzhou, 310020 (CN); MA, Jingxuan, Hangzhou, 310020 (CN); MA, Yuqing, Hangzhou, 310020 (CN); JIN, Lijie, Hangzhou, 310020 (CN); YU, Fei, Hangzhou, 310020 (CN)
(74) Representative: Dehns

(57) **Abstract**

The present invention discloses a Toll-like receptor agonist-based vaccine adjuvant lipid compound and use thereof, and specifically discloses a compound of formula (I), or a stereoisomer, an N-oxide, a solvate, or a pharmaceutically acceptable salt thereof. The mRNA-TLP composition prepared from the adjuvant lipid compound of the present disclosure significantly enhances targeting to antigen-presenting cells in mouse spleen and can significantly inhibit tumor growth.

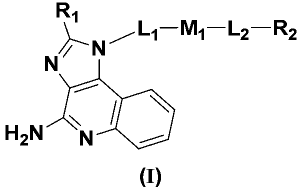

## Description

The present application claims priority from Chinese Patent Application No. 202411874747.X filed on December 19, 2024.

### TECHNICAL FIELD

The present disclosure relates to a Toll-like receptor agonist-based vaccine adjuvant lipid compound and use thereof.

### BACKGROUND

In recent years, messenger ribonucleic acid (mRNA)-based tumor immunotherapies have attracted extensive attention due to their significant clinical potential. Compared with DNA vaccines, mRNA can induce transient expression of tumor antigens while avoiding the possibility of insertional mutagenesis. In addition, mRNA can enhance therapeutic efficacy by inducing stronger humoral and cellular responses. Compared with traditional protein-based vaccines, mRNA vaccines exhibit numerous advantages, such as biocompatibility, non-toxicity, as well as simple and scalable production processes. Inspired by the advantages of mRNA cancer vaccines, more than twenty mRNA-based immunotherapies have demonstrated antitumor potential in preclinical and clinical studies.

To reduce the high innate immunogenicity and improve tolerance and translation efficiency, modified nucleosides such as 1-methylpseudouridine (m1ψ) have been incorporated into mRNA sequences transcribed *in vitro.* However, such modification raises the concern that it may impair innate immunogenicity. Immunogenicity is essential for activating dendritic cells (DCs), which are the primary antigen-presenting cells.

Toll-like receptors (TLRs), as a family of transmembrane proteins, are innate immune receptors directly or indirectly responsible for detecting pathogen-associated molecular patterns (PAMPs). TLRs can initiate a series of biosynthetic reactions by activating DCs, including the production of specific cytokines (*e*.*g*., tumor necrosis factor-α (TNF-α)), which enhances the expression of co-stimulatory molecules and improves antigen-presenting capacity. These molecular mechanisms are essential for activating both innate and adaptive immune responses, effectively stimulating the transition from innate to adaptive immunity. The incorporation of lipid-modified TLRs into mRNA delivery systems to enhance the magnitude and duration of adaptive immune responses has been increasingly studied. Therefore, developing a new generation of vaccine adjuvant lipids based on Toll-like receptor 7 and 8 dual agonists is of great significance for improving mRNA-based cancer therapy.

### SUMMARY

The technical problem addressed by the present disclosure is to provide a Toll-like receptor agonist-based vaccine adjuvant lipid compound and use thereof in order to overcome the structural monotony of vaccine adjuvant lipid compounds based on Toll-like receptor 7 and 8 dual agonists and the poor targeting of antigen-presenting cells by the resulting mRNA compositions in the prior art. The mRNA-TLP composition prepared from the adjuvant lipid compound of the present disclosure significantly enhances targeting to antigen-presenting cells in mouse spleen and can significantly inhibit tumor growth.

The present disclosure provides a compound of formula (I), or a stereoisomer, an N-oxide, a solvate, or a pharmaceutically acceptable salt thereof: 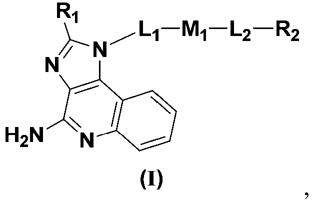
L₁ is C₁₋₁₀ alkylene or C₁₋₄ alkylene-C₆₋₁₀ aryl-C₁₋₄ alkylene;
M₁ is -NH-, -OC(O)HN-, -C(O)O-, or -O-;
L₂ is C₁₋₈ alkylene, -R₃ₐC(O)OR₄ₐ-, -R_{3b}OR_{4b}-, 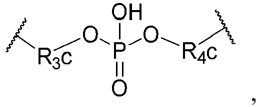 or 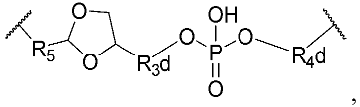 wherein R₃ₐ, R_{3b}, R_{3c}, R_{3d}, R₄ₐ, R_{4b}, R_{4c}, R_{4d}, and R₅ are independently C₁₋₈ alkylene;
R₁ is C₁₋₅ alkyl or C₁₋₅ alkyl substituted by C₁₋₆ alkoxy;
R₂ is 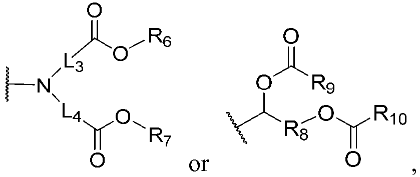
wherein L₃ and L₄ are independently C₁₋₇ alkylene;
R₆ and R₇ are independently C₇₋₂₈ alkyl;
R₈ is C₁₋₇ alkylene, and R₉ and R₁₀ are independently C₁₀₋₂₀ alkyl or C₁₀₋₂₀ alkenyl.

In some embodiments, L₁ is C₁₋₆ linear alkylene, and L₂ is C₁₋₆ linear alkylene-C(O)O-C₁₋₄ linear alkylene^{a} or 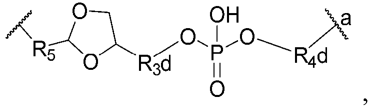 wherein the a-terminus is connected to R²; R_{3d}, R_{4d}, and R₅ are independently C₁₋₄ linear alkylene;
or, L₁ is C₁₋₄ linear alkylene-C₆₋₁₀ aryl-C₁₋₄ linear alkylene, and L₂ is C₁₋₄ linear alkylene or C₁₋₆ linear alkylene-C(O)O-C₁₋₄ linear alkylene^{a}, wherein the a-terminus is connected to R².

In some embodiments, R₁ is C₁₋₅ linear alkyl.

In some embodiments, L₃ is C₁₋₄ linear alkylene.

In some embodiments, L₄ is C₄₋₆ linear alkylene.

In some embodiments, R₆ is C₈₋₁₂ linear alkyl.

In some embodiments, R₇ is C₁₆₋₂₀ branched alkyl.

In some embodiments, R₈ is C₁₋₄ linear alkylene.

In some embodiments, R₉ and R₁₀ are independently C₁₆₋₂₀ alkenyl.

In some embodiments, L₁ is -(CH₂)₄-, -CH₂C((CH₃)₂)-^{b}, or 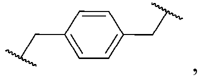 wherein the b-terminus is connected to M₁.

In some embodiments, Mi is -NH- or -OC(O)HN-, wherein the N-terminus is connected to L₂.

In some embodiments, Mi is -NH-.

In some embodiments, L₂ is -(CH₂)₂-, -(CH₂)₃C(O)O(CH₂)₂-^{a}, -(CH₂)₅C(O)O(CH₂)₂-a , 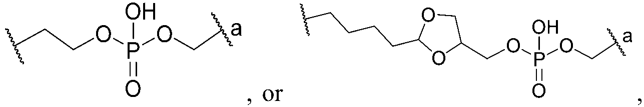 wherein the a-terminus is connected to R₂.

In some embodiments, L₁ is -(CH₂)₄-, and L₂ is -(CH₂)₅C(O)O(CH₂)₂-^{a} or 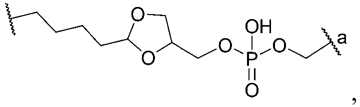 wherein the a-terminus is connected to R₂;
or, L₁ is 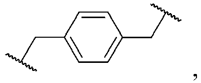 and L₂ is -(CH₂)₂-, -(CH₂)₃C(O)O(CH₂)₂-^{a}, or - (CH₂)₅C(O)O(CH₂)₂-^{a}, wherein the a-terminus is connected to R₂.

In some embodiments, R₁ is -(CH₂)₃CH₃ or -CH₂OCH₂CH₃.

In some embodiments, R₂ is 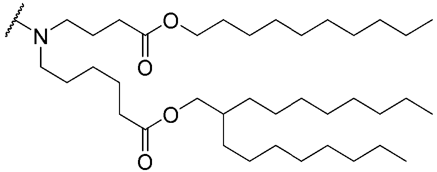 or

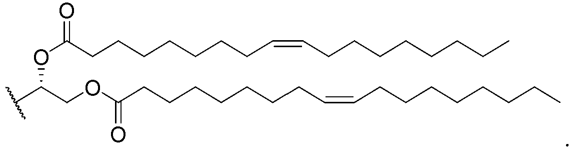

In some embodiments, the compound of formula (I) is any one of the following compounds: 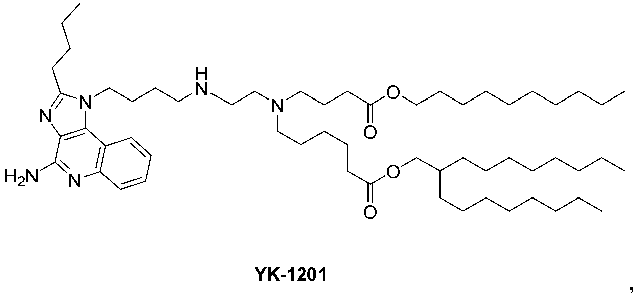 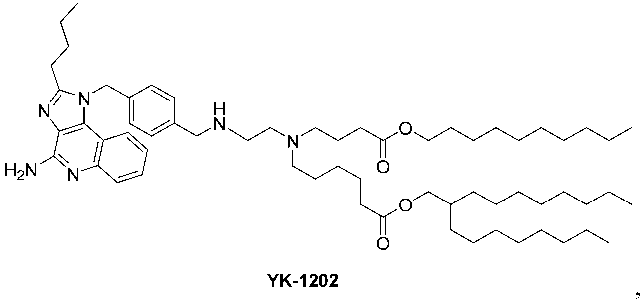 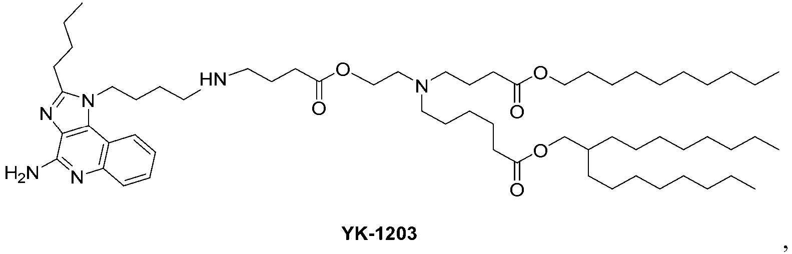 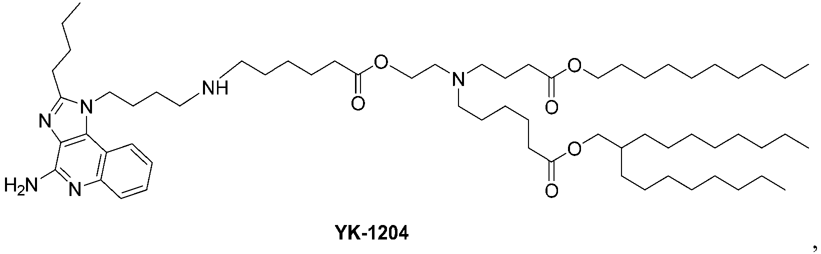 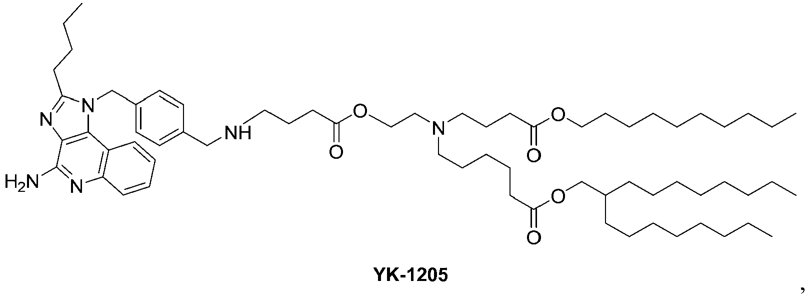 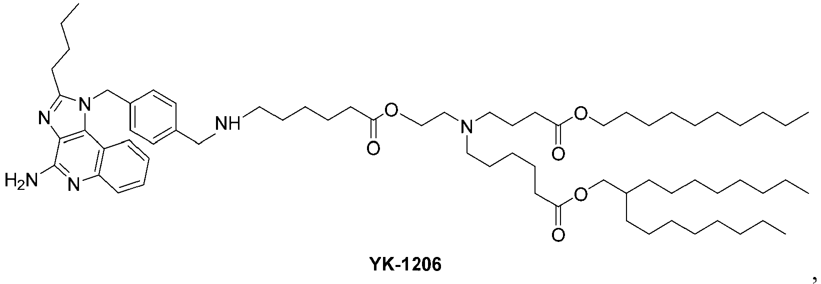 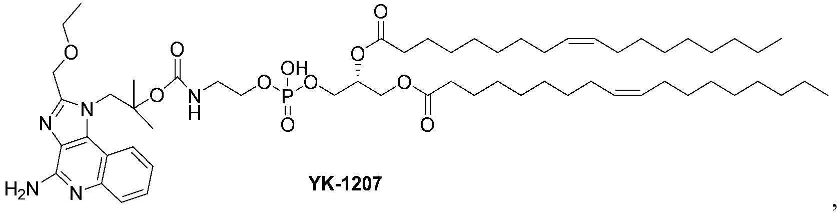 or

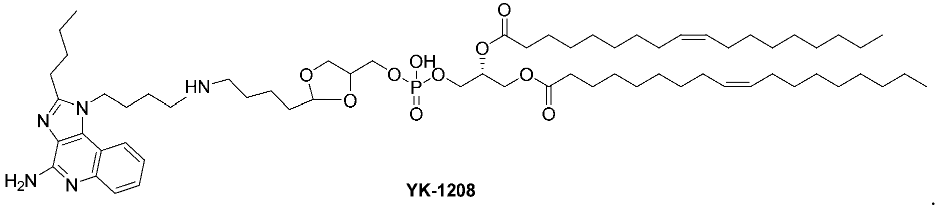

The present disclosure provides a lipid composition, comprising a substance Z, wherein the substance Z is a compound of formula (I) as described above, or a stereoisomer, an N-oxide, a solvate, or a pharmaceutically acceptable salt thereof.

In some embodiments, the molar percentage of the substance Z in the lipid composition is 2.5 to 20%.

In some embodiments, the molar percentage of the substance Z in the lipid composition is 5 to 15%.

In some embodiments, the molar percentage of the substance Z in the lipid composition is 10%.

In some embodiments, the lipid composition comprises the substance Z, a permanent anionic lipid, a permanent cationic lipid, and a neutral lipid. The molar percentage ratio of the substance Z, the permanent anionic lipid, the permanent cationic lipid, and the neutral lipid is preferably (2.5 to 20):(10 to 33):(20 to 60):(20 to 40).

In some embodiments, the permanent anionic lipid is any one selected from the group consisting of 2-acetamidoethyl ((R)-2,3-bis(oleoyloxy)propyl) phosphate, (R)-2,3-bis(oleoyloxy)propyl-(2-(3-ethylthioureido)ethyl) phosphate, (R)-2,3-bis(oleoyloxy)propyl-(2-(3-ethylureido)ethyl) phosphate, (R)-2,3-bis(oleoyloxy)propyl-(2-(3-propylureido)ethyl) phosphate, (R)-2,3-bis(oleoyloxy)propyl-(2-(3-butylureido)ethyl) phosphate, and salts thereof, or any combination of at least two thereof.

In some embodiments, the permanent anionic lipid is sodium 2-acetamidoethyl ((R)-2,3-bis(oleoyloxy)propyl) phosphate.

In some embodiments, the molar percentage of the permanent anionic lipid in the lipid composition is 25%.

In some embodiments, the permanent cationic lipid is any one selected from the group consisting of 1,2-di-O-octadecenyl-3-trimethylammonium propane, (2,3-dioleoyloxypropyl)trimethylammonium, and salts thereof, or any combination of at least two thereof.

In some embodiments, the permanent cationic lipid is 1,2-di-O-octadecenyl-3-trimethylammonium propane (chloride salt).

In some embodiments, the molar percentage of the permanent cationic lipid in the lipid composition is 30 to 47.5%, such as 35%, 40%, or 45%.

In some embodiments, the neutral lipid is any one selected from the group consisting of 1,2-dioleoyl-sn-glycero-3-phosphoethanolamine, 1,2-distearoyl-sn-glycero-3-phosphocholine, and salts thereof, or any combination of at least two thereof.

In some embodiments, the neutral lipid is 1,2-dioleoyl-sn-glycero-3-phosphoethanolamine.

In some embodiments, the molar percentage of the neutral lipid in the lipid composition is 25%.

In some embodiments, the lipid composition comprises the substance Z, 2-acetamidoethyl ((R)-2,3-bis(oleoyloxy)propyl) phosphate, 1,2-di-O-octadecenyl-3-trimethylammonium propane (chloride salt), and 1,2-dioleoyl-sn-glycero-3-phosphoethanolamine; wherein the molar percentage ratio of the substance Z, 2-acetamidoethyl ((R)-2,3-bis(oleoyloxy)propyl) phosphate, 1,2-di-O-octadecenyl-3-trimethylammonium propane (chloride salt), and 1,2-dioleoyl-sn-glycero-3-phosphoethanolamine is (2.5 to 20):25:(30 to 47.5):25; preferably (5 to 15):25:(35 to 45):25, and more preferably 10:25:40:25.

In some embodiments, the lipid composition comprises the substance Z, a cationic lipid, a neutral lipid, a structured lipid, and a polymer-conjugated lipid. The molar percentage ratio of the substance Z, the cationic lipid, the neutral lipid, the structured lipid, and the polymer-conjugated lipid is preferably (2.5 to 20):(25 to 75):(5 to 25):(15 to 65):(0.5 to 10).

In some embodiments, the cationic lipid is any one selected from the group consisting of the following compounds in (1) to (7), or any combination of at least two thereof:
(1) a compound of formula (II), or a stereoisomer, an N-oxide, a solvate, or a pharmaceutically acceptable salt thereof, wherein G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl; 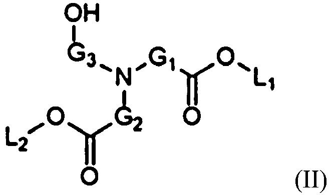
(2) a compound of formula (III), or a stereoisomer, an N-oxide, a solvate, or a pharmaceutically acceptable salt thereof, wherein G₁ is C₂₋₈ alkylene; G₂ is C₂₋₈ alkylene; L₁ is -C(O)O- or -OC(O)-; L₂ is -C(O)O- or -OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or -(CH₂)₄-; 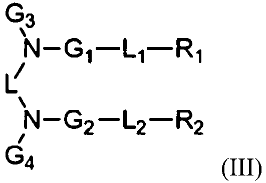
(3) a compound of formula (IV), or a stereoisomer, an N-oxide, a solvate, or a pharmaceutically acceptable salt thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-; 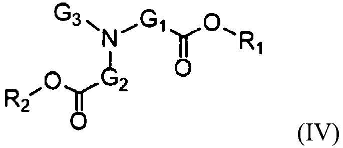
(4) a compound of formula (V), or a stereoisomer, an N-oxide, a solvate, or a pharmaceutically acceptable salt thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is -CH₃, -CH₂CH₃, or -CH₂CH₂OH; 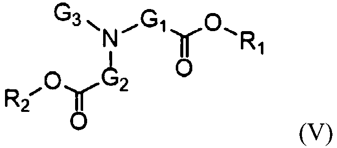
(5) a compound of formula (VI), or a stereoisomer, an N-oxide, a solvate, or a pharmaceutically acceptable salt thereof, wherein G¹ and G² are each independently C₆₋₁₀ alkylene; G³ is C₁₋₁₂ alkylene; R¹ and R² are each independently C₆₋₂₄ alkyl or C₆₋₂₄ alkenyl; R³ is OR⁵, N, -C(=O)OR⁴, -OC(=O)R⁴, or -NR⁵C(=O)R⁴; R⁴ is C₁₋₁₂ hydrocarbyl; R⁵ is H or C₁₋₆ hydrocarbyl; 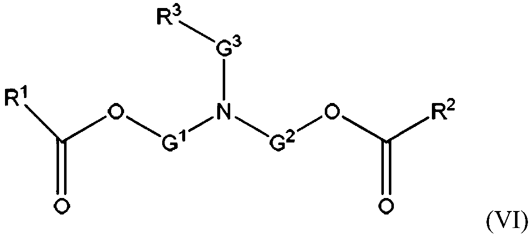
(6) a compound of formula (VII), or a stereoisomer, an N-oxide, a solvate, or a pharmaceutically acceptable salt thereof, wherein R₄ is -(CH₂)ₙQ or -(CH₂)ₙCHQR; Q is -OR, -OH, -O(CH₂)ₙN(R)₂, -OC(O)R, -CX₃, -CN, -N(R)C(O)R, -N(H)C(O)R, -N(R)S(O)₂R, - N(H)S(O)₂R, -N(R)C(O)N(R)₂, -N(H)C(O)N(R)₂, -N(H)C(O)N(H)(R), -N(R)C(S)N(R)₂, - N(H)C(S)N(R)₂, -N(H)C(S)N(H)(R), -N(R)S(O)₂R, or heterocycle; n is 1, 2, or 3; R is C₁₋₈ alkyl; X is H or C₁₋₈ alkyl; 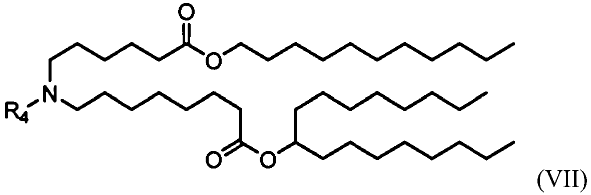
(7) a compound of formula (VIII), or a stereoisomer, an N-oxide, a solvate, or a pharmaceutically acceptable salt thereof,

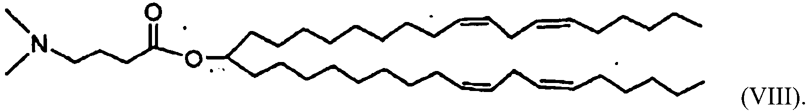

In some embodiments, the cationic lipid is any one selected from the group consisting of YK-009, YK-401, YK-305, ALC0315, SM102, and DLIN-MC3-DMA, or any combination of at least two thereof:

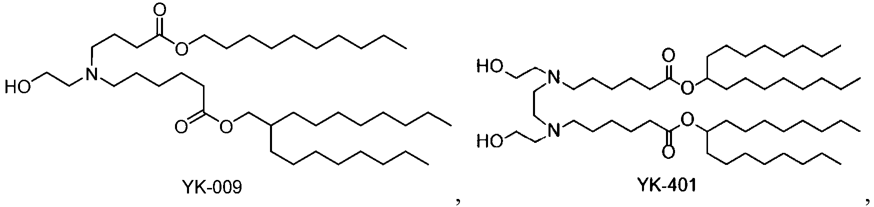

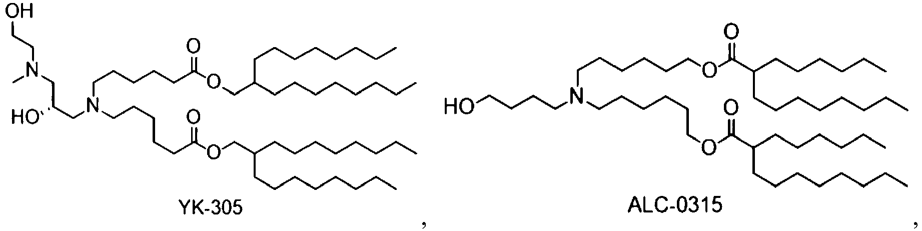

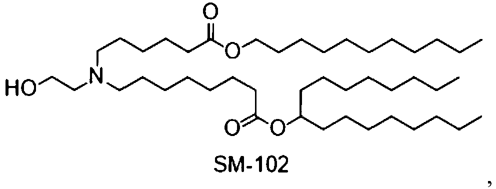

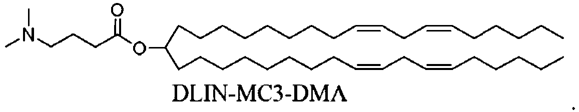

In some embodiments, the cationic lipid is YK-009.

In some embodiments, the molar percentage of the cationic lipid in the lipid composition is 30 to 47.5%, such as 35%, 40%, or 45%.

In some embodiments, the neutral lipid is any one selected from the group consisting of phosphatidylcholine, phosphatidylethanolamine, sphingomyelin, ceramide, sterol, and derivatives thereof, or any combination of at least two thereof.

In some embodiments, the neutral lipid is any one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, and lysophosphatidylethanolamine (LPE), or any combination of at least two thereof.

In some embodiments, the neutral lipid is DOPE and/or DSPC, preferably DSPC.

In some embodiments, the molar percentage of the neutral lipid in the lipid composition is 10%.

In some embodiments, the structured lipid is any one selected from the group consisting of cholesterol, nonsterol, sitosterol, ergosterol, campesterol, stigmasterol, brassicasterol, tomatine, ursolic acid, α-tocopherol, and corticosteroid, or any combination of at least two thereof.

In some embodiments, the structured lipid is cholesterol.

In some embodiments, the molar percentage of the structured lipid in the lipid composition is 38.5%.

In some embodiments, the polymer-conjugated lipid is any one selected from the group consisting of distearoyl phosphatidylethanolamine polyethylene glycol 2000 (DSPE-PEG2000), 1,2-dimyristoyl-sn-glycero-3-methoxypolyethylene glycol 2000 (DMG-PEG2000), and methoxypolyethylene glycol ditetradecylacetamide (ALC-0159), or any combination of at least two thereof.

In some embodiments, the polymer-conjugated lipid is DMG-PEG2000.

In some embodiments, the molar percentage of the polymer-conjugated lipid in the lipid composition is 1.5%.

In some embodiments, the lipid composition comprises the substance Z, the YK-009, DSPC, cholesterol, and DMG-PEG2000; wherein the molar percentage ratio of the substance Z, the YK-009, DSPC, cholesterol, and DMG-PEG2000 is preferably (2.5 to 20):(30 to 47.5):10:38.5:1.5; more preferably 10:40:10:38.5:1.5.

In some embodiments, the lipid composition further comprises one or more cell-penetrating peptides.

The present disclosure provides a pharmaceutical composition, comprising: (A) a therapeutic and/or prophylactic agent, comprising any one of the group consisting of a nucleic acid molecule, a small molecule compound, a polypeptide, or a protein, or any combination of at least two thereof;

(B) the lipid composition as described above.

In some embodiments, the therapeutic and/or prophylactic agent and the lipid composition are used in an amount such that the charge ratio of positive to negative charges in the pharmaceutical composition is 1:(2 to 5), such as 1:2.

In some embodiments, the mass ratio of the lipid composition to the therapeutic or prophylactic agent is (12.5 to 25):1.

In some embodiments, the mass ratio of the lipid composition to the therapeutic or prophylactic agent is 15:1.

In some embodiments, the pharmaceutical composition is used for delivering the therapeutic and/or prophylactic agent to antigen-presenting cells in a target organ or tissue.

In some embodiments, the target organ or tissue is any one selected from the group consisting of spleen, liver, lymph, muscle, and lung, or any combination of at least two thereof; preferably spleen or lymph.

In some embodiments, the antigen-presenting cell is any one selected from the group consisting of B cells, NK cells, cDCs, pDCs, and macrophages, or any combination of at least two thereof.

In some embodiments, the therapeutic and/or prophylactic agent is a nucleic acid molecule capable of encoding one or more antigens.

In some embodiments, the antigen is a disease-associated antigen, or the nucleic acid molecule or antigen is capable of eliciting an immune response against the disease-associated antigen or a cell expressing the disease-associated antigen.

In some embodiments, the nucleic acid molecule is an RNA encoding one or more antigens.

In some embodiments, the pharmaceutical composition further comprises at least one auxiliary ingredient. The auxiliary ingredient may be a pharmaceutically acceptable carrier, diluent, or excipient.

In some embodiments, the pharmaceutical composition further comprises one or more hydrophobic small molecules, permeability-enhancing molecules, carbohydrates, polymers, surface-altering agents, functionalized lipids, or cytokines.

The present disclosure also provides use of the substance Z as described above, the lipid composition as described above, or the pharmaceutical composition as described above in the manufacture of a nucleic acid drug, a gene vaccine, a small molecule drug, or a polypeptide or protein drug.

The present disclosure also provides use of the substance Z as described above, the lipid composition as described above, or the pharmaceutical composition as described above in the manufacture of a medicament for treating a disease or disorder. The disease or disorder is preferably characterized by dysfunctional or abnormal protein or polypeptide activity.

In some embodiments, the disease or disorder is any one selected from the group consisting of infectious disease, tumor, proliferative disease, genetic disease, autoimmune disease, diabetes, neurodegenerative disease, cardiovascular and renal vascular disease, and metabolic disease, or any combination of at least two thereof.

In some embodiments, the infectious disease is a disease caused by coronavirus, influenza virus, or HIV, pediatric pneumonia, Rift Valley fever, yellow fever, rabies, or multiple types of herpes.

In some embodiments, the tumor is breast cancer, ovarian cancer, lung cancer, pancreatic cancer, kidney cancer, gastric cancer, lymphoma, colon cancer, liver cancer, melanoma, bladder cancer, cervical cancer, or prostate cancer.

In some embodiments, in the use, the medicament is used for treating a disease or disorder in a mammal in need thereof. The mammal may be any one selected from the group consisting of humans, non-human primates, companion animals, exotic species, livestock animals, and food animals, or any combination of at least two thereof.

In some embodiments, the medicament is administered intravenously, intramuscularly, intradermally, subcutaneously, intranasally, or by inhalation.

In some embodiments, the medicament is administered intravenously or intramuscularly.

In some embodiments, the medicament is administered at a dose of 0.001 to 10 mg/kg.

The adjuvant lipid compound and lipid composition provided by the present disclosure may be used for nucleic acid (*e*.*g*., mRNA) encapsulation to form corresponding nucleic acid drugs.

### Definition of terms

All publications and patents referenced in the present disclosure are incorporated herein by reference in their entirety. If usage or terminology used in any publications and patents incorporated by reference conflicts with usage or terminology used in the present disclosure, the usage and terminology in the present disclosure shall prevail.

The section headings used herein are for the sole purpose of organizing the article and should not be construed as a limitation on the subject matter described.

Unless otherwise specified, all technical and scientific terms used herein have their ordinary meanings in the art to which the claimed subject matter pertains. If there are multiple definitions for a term, the definition herein shall prevail.

Except in the examples or otherwise indicated, all numbers stating quantitative properties, such as doses, in the specification and claims should be understood as modified in all instances by the term "about". It should also be understood that any numerical range recited in the present disclosure is intended to include all sub-ranges within the range and any combination of the various endpoints of the range or sub-ranges.

In the present disclosure, " 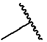 " in a structural fragment indicates that the structural fragment is connected to the rest of the molecule via the bond. For example, 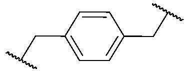 indicates that it is connected to the rest of the molecule via " 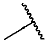 "

In the present disclosure, the term "one or more" refers to 1, 2, 3, 4, 5, or 6, such as 1, 2, or 3.

In the present disclosure, the term "alkyl" refers to a linear or branched, saturated monovalent hydrocarbon group having a specified number of carbon atoms (*e.g*., C₁, C₂, C₃, C₄, C₅, C₆, C₉, C₁₀, C₁₁, C₁₂, C₁₆, C₁₇, C₁₈, C₁₉, C₂₀). Alkyl includes, but is not limited to, methyl, ethyl, n-propyl, isopropyl, n-butyl, isobutyl, sec-butyl, *tert*-butyl, n-pentyl, n-hexyl, 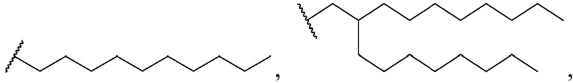 *etc.*

In the present disclosure, the term "alkylene" refers to a saturated divalent hydrocarbon group obtained by removing two hydrogen atoms from a saturated linear or branched hydrocarbon group. Alkylene includes, but is not limited to, methylene (-CH₂-), ethylene (including -CH₂CH₂- or -CH(CH₃)-), isopropylene (including -CH(CH₃)CH₂- or - C(CH₃)₂-), 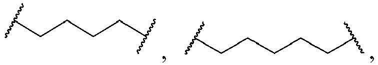 *etc.*

In the present disclosure, the term "alkoxy" refers to a group R^{Y}-O-, wherein R^{Y} is as defined in the term "alkyl". Alkoxy includes, but is not limited to, methoxy, ethoxy, *n-*propoxy, isopropoxy, *n*-butoxy, isobutoxy, *sec*-butoxy, *tert*-butoxy, *n*-pentoxy, *n*-hexoxy, *etc.*

The term "alkenyl" refers to a linear or branched hydrocarbon group having at least one double bond, consisting solely of carbon and hydrogen atoms, having, for example, 10 to 20 (for another example, 16, 17, 18, or 19) carbon atoms, and connected to the rest of the molecule via a single bond. Alkenyl includes, but is not limited to, vinyl, 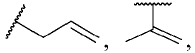 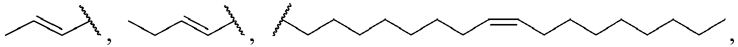 *etc.*

In the present disclosure, the term "aryl" refers to a cyclic, unsaturated monovalent hydrocarbon group having a specified number of carbon atoms (*e.g.,* C₆-C₁₀), which is monocyclic or polycyclic (*e*.*g*., bicyclic), wherein the rings share two atoms and one bond when polycyclic, and each ring is aromatic. The aryl group is connected to the rest of the molecule via an aromatic ring. Aryl includes, but is not limited to, phenyl and naphthyl.

Furthermore, when referring to a number or numerical range, the term "about" means that the referenced number or numerical range is an approximation within the typical tolerance range in the art, within the experimental variability, or within the statistical experimental error, and thus the number or numerical range may vary, for example, between 1% and 15% of the stated number or numerical range. For example, "about" may be understood as approximately 2 standard deviations of the mean. When "about" precedes a series of numbers or a range, it should be understood that "about" may modify each number in the series or range.

Additionally, in the present disclosure, when a numerical range is used in a general formula and/or structural formula of a compound, it means that the number of corresponding groups in the numerical range may be any natural number within the numerical range. For example, "C_{A-B}" means that the number of carbon atoms is any integer from the starting point to the ending point, wherein both A and B are integers. For another example, C₁₋₅ means that the number of carbon atoms is 1, 2, 3, 4, or 5. That is, when combined with other groups in the general formula and/or structural formula of the compound to form various possible compounds, C_{A-B} may be used in combination with any carbon-containing group to define the number of carbon atoms. For example, C₁₋₅ alkyl/alkylene represents various possibilities of alkyl/alkylene with 1 C, 2 C, 3 C, 4 C, and/or 5 C.

The words "including", "containing", or "comprising" and the like as used herein mean that the element appearing before the word covers the elements listed after the word and their equivalents, and does not exclude unrecited elements. The term "containing" or "including (comprising)" as used herein may be open, semi-closed, or closed. In other words, the term also includes "consisting essentially of" or "consisting of'.

The term "pharmaceutically acceptable" as used herein means that a compound or composition is chemically and/or toxicologically compatible with the other ingredients making up the preparation and/or with the human or mammal in which it is used to prevent or treat a disease or disorder.

The term "subject" or "patient" as used herein may include mammalian subjects. For example, the mammal subject may be any one selected from the group consisting of humans, non-human primates, companion animals, exotic species, livestock animals, and food animals, or any combination of at least two thereof.

The term "treatment" as used herein refers to the administration of one or more pharmaceutical substances to a patient or subject suffering from a disease or exhibiting symptoms of the disease, for the purpose of curing, alleviating, relieving, improving, or affecting the disease or the symptoms of the disease. In the context of the present disclosure, unless specifically stated to the contrary, the term "treatment" may also include prevention.

In the present disclosure, the term "antigen" includes any molecule, preferably a peptide or protein, containing at least one epitope capable of eliciting an immune response and/or an epitope targeted by an immune response. Preferably, the antigen in the context of the present disclosure is a molecule which, optionally after processing, induces an immune response that is preferably specific to the antigen or a cell expressing the antigen. Specifically, "antigen" refers to a molecule which, optionally after processing, is presented by MHC molecules and specifically reacts with T lymphocytes (T cells).

Thus, the antigen or a fragment thereof should be capable of being recognized by a T cell receptor. Preferably, if recognized by a T cell receptor, an antigen or fragment is capable of inducing clonal expansion of T cells carrying a T cell receptor that specifically recognizes the antigen or fragment in the presence of a suitable co-stimulatory signal. In the context of embodiments of the present disclosure, an antigen or fragment is preferably presented by a cell, preferably an antigen-presenting cell and/or a diseased cell in the context of MHC molecules, which results in an immune response against the antigen or a cell expressing the antigen.

According to the present disclosure, any suitable antigen is contemplated as a candidate for an immune response, wherein the immune response is preferably a cellular immune response.

The antigen is preferably a product corresponding to or derived from a naturally occurring antigen. The naturally occurring antigen may include or may be derived from allergens, viruses, bacteria, fungi, parasites, and other infectious agents and pathogens, or the antigen may also be a tumor antigen. According to the present disclosure, the antigen may correspond to a naturally occurring product, such as a viral protein or a portion thereof.

The term "pathogen" refers to pathogenic microorganisms and includes viruses, bacteria, fungi, unicellular organisms, and parasites. Examples of pathogenic viruses include, but are not limited to, human immunodeficiency virus (HIV), cytomegalovirus (CMV), herpes simplex virus (HSV), hepatitis A virus (HAV), HBV, HCV, papillomavirus, and human T-lymphotrophic virus (HTLV). Unicellular organisms include, but are not limited to, Plasmodium, Trypanosoma, Amoebae, *etc.*

The term "disease-associated antigen" refers to all antigens with pathogenic significance and includes "tumor antigens". According to the present disclosure, it is desirable to induce an immune response against a disease-associated antigen or a cell expressing the disease-associated antigen and preferably presenting the disease-associated antigen in the context of MHC molecules. Preferably, the disease-associated antigen is a naturally occurring antigen. In one embodiment, the disease-associated antigen is expressed in diseased cells and is preferably presented by cellular MHC molecules.

The antigen encoded by the RNA (*i.e*., the therapeutic and/or prophylactic agent) contained in the nanoparticle (lipid composition) according to the present disclosure should induce an immune response against a disease-associated antigen to be targeted or a cell expressing the disease-associated antigen to be targeted. Thus, the antigen encoded by the RNA contained in the nanoparticle according to the present disclosure may correspond to or may comprise a disease-associated antigen or one or more immunogenic fragments thereof, such as one or more MHC-binding peptides of the disease-associated antigen. Accordingly, the antigen encoded by the RNA contained in the nanoparticle according to the present disclosure may be a recombinant antigen.

### Therapeutic and/or prophylactic agent

The lipid composition of the present disclosure may be used to deliver an active pharmaceutical ingredient, such as a therapeutic and/or prophylactic agent. Based on the above, the present disclosure further provides a (pharmaceutical) composition comprising the lipid composition provided by the present disclosure for delivering an active pharmaceutical ingredient. The composition of the present disclosure may comprise one or more therapeutic and/or prophylactic agents (as active pharmaceutical ingredients). The active pharmaceutical ingredient may be encapsulated within the lipid composition or associated with the lipid composition.

The therapeutic and/or prophylactic agent includes, but is not limited to, one or more of a nucleic acid molecule, a small molecule compound, a polypeptide, and a protein. Preferably, the therapeutic and/or prophylactic agent is a nucleic acid molecule.

For example, the therapeutic and/or prophylactic agent is a vaccine or compound capable of eliciting an immune response. Thus, in some preferred embodiments, the therapeutic and/or prophylactic agent may be a nucleic acid molecule capable of encoding one or more antigens.

The lipid composition of the present disclosure may deliver the therapeutic and/or prophylactic agent to target cells and/or target organs in a subject (*e.g.,* a mammal) (as a carrier). Thus, the present disclosure also provides a method for treating a disease or disorder in a subject in need thereof, comprising administering to the subject a composition comprising the therapeutic and/or prophylactic agent and/or contacting cells of the subject with the composition.

The therapeutic and/or prophylactic agent comprises a biologically active substance and may alternatively be referred to as an "active agent", "active ingredient", or the like. The therapeutic and/or prophylactic agent may be a substance that, upon delivery to a cell or organ, induces a desired change in the cell or organ or in other body tissues or systems. Such substances may be used to treat one or more diseases, disorders, or conditions. In some embodiments, the therapeutic and/or prophylactic agent is a small molecule drug for treating a specific disease, disorder, or condition. Examples of drugs suitable for use in the composition include, but are not limited to, antineoplastic agents (*e*.*g*., vincristine, doxorubicin, mitoxantrone, camptothecin, cisplatin, bleomycin, cyclophosphamide, methotrexate, and streptozotocin), antitumor agents (*e*.*g*., actinomycin D, vincristine, vinblastine, cytosine arabinoside, anthracycline, alkylating agents, platinum compounds, antimetabolites, and nucleoside analogs, such as methotrexate and purine and pyrimidine analogs), anti-infective agents, local anesthetics (*e.g.,* dibucaine and chlorpromazine), β-adrenergic blockers (*e.g*., propranolol, timolol, and labetalol), antihypertensives (*e*.*g*., clonidine and hydralazine), antidepressants (*e.g.,* imipramine, amitriptyline, and doxepin), antispasmodics (*e.g*., phenytoin), antihistamines (*e*.*g*., diphenhydramine, chlorpheniramine, and promethazine), antibiotics/antibacterial agents (*e*.*g*., gentamycin, ciprofloxacin, and cefoxitin), antifungal agents (*e*.*g*., miconazole, terconazole, econazole, isoconazole, butaconazole, clotrimazole, itraconazole, nystatin, naftifine, and amphotericin B), antiparasitic agents, hormones, hormone antagonists, immunomodulators, neurotransmitter antagonists, antiglaucoma agents, vitamins, sedatives, and imaging agents.

In some embodiments, the therapeutic and/or prophylactic agent is a cytotoxin, a radioactive ion, a chemotherapeutic agent, a vaccine, a compound that elicits an immune response, and/or another therapeutic and/or prophylactic agent. Cytotoxins or cytotoxic agents include any agent that is detrimental to cells. Examples include, but are not limited to, taxol, cytochalasin B, gramicidin D, ethidium bromide, emetine, mitomycin, etoposide, teniposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids such as maytansinol, rachelmycin (CC-1065), and analogs or homologs thereof. Radioactive ions include, but are not limited to, iodine (*e*.*g*., iodine-125 or iodine-131), strontium-89, phosphorus, palladium, cesium, iridium, phosphate, cobalt, yttrium-90, samarium-153, and praseodymium. Vaccines include compounds and preparations capable of providing immunity against one or more conditions associated with infectious diseases such as influenza, measles, human papillomavirus (HPV), rabies, meningitis, pertussis, tetanus, plague, hepatitis, and tuberculosis, and may include nucleic acid molecules (*e*.*g*., mRNA) encoding antigens and/or epitopes derived from infectious diseases. Vaccines may also include compounds and preparations that elicit an immune response against cancer cells and may include nucleic acid molecules (*e.g.,* mRNA) encoding antigens, epitopes, and/or neo-epitopes derived from tumor cells. Compounds that elicit an immune response may include vaccines, corticosteroids (*e*.*g*., dexamethasone), and other substances. Other therapeutic and/or prophylactic agents include, but are not limited to, antimetabolites (*e*.*g*., methotrexate, 6-mercaptopurine, 6-thioguanine, cytarabine, and 5-fluorouracil dacarbazine), alkylating agents (*e*.*g*., mechlorethamine, thiotepa, chlorambucil, rachelmycin (CC-1065), melphalan, carmustine (BSNU), lomustine (CCNU), cyclophosphamide, busulfan, dibromomannitol, streptozotocin, mitomycin C, and cis-dichlorodiammineplatinum(II) (DDP), cisplatin), anthracyclines (*e*.*g*., daunorubicin (formerly known as daunomycin) and doxorubicin), antibiotics (*e*.*g*., dactinomycin (formerly known as actinomycin), bleomycin, mithramycin, and anthramycin (AMC)), and antimitotic agents (*e*.*g*., vincristine, vinblastine, taxol, and maytansinoids).

In other embodiments, the therapeutic and/or prophylactic agent is a protein. Therapeutic proteins suitable for use in the nanoparticles of the present disclosure include, but are not limited to, gentamicin, amikacin, insulin, erythropoietin (EPO), granulocyte colony-stimulating factor (G-CSF), granulocyte-macrophage colony-stimulating factor (GM-CSF), factor VIR, luteinizing hormone-releasing hormone (LHRH) analogs, interferons, heparin, hepatitis B surface antigen, typhoid vaccine, and cholera vaccine.

In some embodiments, the therapeutic and/or prophylactic agent may be a polynucleotide or nucleic acid (*e*.*g*., ribonucleic acid or deoxyribonucleic acid). The term "polynucleotide" in its broadest sense includes any compound and/or substance present in an oligonucleotide chain or capable of being incorporated into an oligonucleotide chain. Exemplary polynucleotides for use according to the present disclosure include, but are not limited to, one or more of the following: deoxyribonucleic acid (DNA); ribonucleic acid (RNA), including messenger mRNA (mRNA) and hybrids thereof; RNAi-inducing agents; RNAi agents; siRNA; shRNA; miRNA; antisense RNA; ribozymes; catalytic DNA; RNA inducing triplex formation; aptamers; and the like. In some preferred embodiments, the therapeutic and/or prophylactic agent is an RNA. The RNA suitable for use in the compositions and methods described herein may be selected from, but is not limited to, the group consisting of: shortmer, antagomir, antisense RNA, ribozyme, small interfering RNA (siRNA), asymmetric interfering RNA (aiRNA), microRNA (miRNA), Dicer-substrate RNA (dsRNA), small hairpin RNA (shRNA), transfer RNA (tRNA), messenger RNA (mRNA), and mixtures thereof. In certain embodiments, the RNA is mRNA.

In certain embodiments, the therapeutic and/or prophylactic agent is an mRNA. The mRNA may encode any polypeptide of interest, including any naturally or non-naturally occurring or otherwise modified polypeptide. The polypeptide encoded by the mRNA may be of any size and may have any secondary structure or activity. In some embodiments, the polypeptide encoded by the mRNA may have a therapeutic effect when expressed in a cell.

In other embodiments, the therapeutic and/or prophylactic agent is an siRNA. The siRNA can selectively reduce or downregulate the expression of a gene of interest. For example, the siRNA may be selected to silence a gene associated with a specific disease, disorder, or condition upon administration of a composition comprising the siRNA to a subject in need thereof. The siRNA may comprise a sequence that is complementary to an mRNA sequence encoding a gene or protein of interest. In some embodiments, the siRNA may be an immunomodulatory siRNA.

In certain embodiments, the therapeutic and/or prophylactic agent is an sgRNA and/or Cas9 mRNA. The sgRNA and/or Cas9 mRNA may be used as a gene-editing tool. For example, the sgRNA-Cas9 complex may affect mRNA translation of cellular genes.

In some embodiments, the therapeutic and/or prophylactic agent is an shRNA or a vector or plasmid encoding the same. The shRNA may be produced inside a target cell upon delivery of an appropriate construct to the nucleus. Constructs and mechanisms associated with shRNA are well known in the relevant art.

### Disease or disorder

The composition/vector of the present disclosure may deliver a therapeutic and/or prophylactic agent to a subject or patient, thereby achieving treatment and/or prevention of a disease or disorder. The therapeutic and/or prophylactic agent includes, but is not limited to, one or more of a nucleic acid molecule, a small molecule compound, a polypeptide, or a protein. Thus, the composition of the present disclosure may be used in the manufacture of a nucleic acid drug, a gene vaccine, a small molecule drug, or a polypeptide or protein drug. Due to the wide variety of the aforementioned therapeutic and/or prophylactic agents, the composition of the present disclosure may be used to treat or prevent a variety of diseases or disorders.

In one embodiment, the disease or disorder is characterized by dysfunctional or abnormal protein or polypeptide activity.

The agents, compositions, and methods described herein may be used to treat a subject suffering from a disease (*e.g*., a disease characterized by the presence of diseased cells expressing an antigen and presenting an antigenic peptide), or to prevent a subject from developing a disease. Examples of diseases that can be treated and/or prevented encompass all diseases expressing one of the antigens described herein. Particularly preferred diseases are infectious diseases (*e*.*g*., viral diseases) and cancerous diseases. The agents, compositions, and methods described herein may also be used for immunization or vaccination to prevent the diseases described herein.

According to the present disclosure, the term "disease" refers to any pathological state, including infectious diseases and cancerous diseases, particularly those forms of infectious diseases and cancerous diseases described herein.

The disease to be treated according to the present disclosure is preferably an antigen-related disease. According to the present disclosure, "antigen-related disease" or similar expressions mean that the antigen is expressed in cells of a diseased tissue or organ. Expression in cells of a diseased tissue or organ may be elevated compared to that in a healthy tissue or organ. In one embodiment, expression occurs only in diseased tissues, while being suppressed in healthy tissues. According to the present disclosure, the antigen-related disease includes infectious diseases and cancerous diseases, wherein the disease-associated antigens are preferably antigens of infectious agents and tumor antigens, respectively. Preferably, the antigen-related disease is a disease involving cells that express an antigen and present the antigen in the context of MHC molecules, particularly class I MHC molecules.

For example, the disease or disorder is selected from the group consisting of infectious diseases, cancerous and proliferative diseases, genetic diseases, autoimmune diseases, diabetes, neurodegenerative diseases, cardiovascular and renal vascular diseases, and metabolic diseases.

Examples of the infectious diseases include: (i) viral infectious diseases, such as AIDS (HIV), hepatitis A, B, or C, herpes zoster (varicella), German measles (rubella virus), yellow fever, dengue fever, flavivirus, coronavirus, influenza virus, rabies virus, and hemorrhagic infectious disease (Marburg virus or Ebola virus); (ii) bacterial infectious diseases, such as Legionnaires' disease (*Legionella*), gastric ulcer (*Helicobacter*), cholera (*Vibrio*), and infections caused by *Escherichia coli, Staphylococci, Salmonella,* or *Streptococci* (tetanus); (iii) infections caused by protozoan pathogens, such as malaria, sleeping sickness, leishmaniasis, and toxoplasmosis, *i.e.,* infections caused by *Plasmodium, Trypanosoma, Leishmania,* and *Toxoplasma*; or (iv) fungal infections caused by, *e.g., Cryptococcus neoformans, Histoplasma capsulatum, Coccidioides immitis, Blastomyces dermatitidis,* or *Candida albicans.*

The cancer or carcinoma (medical term: malignant tumor) is a class of diseases in which a group of cells exhibits uncontrolled growth (division beyond normal limits), invasion (infiltration and destruction of adjacent tissues), and sometimes metastasis (spread to other parts of the body via lymph or blood). These three malignant attributes distinguish cancer from benign tumors, which are self-limiting and do not invade or metastasize. Most cancers form tumors, *i.e.,* swellings or lesions resulting from abnormal growth of cells (referred to as neoplastic cells or tumor cells), whereas some (*e*.*g*., leukemia) do not. According to the present disclosure, the term "cancer" includes leukemia, seminoma, melanoma, teratoma, lymphoma, sarcoma, blastoma, neuroblastoma, glioma, glioblastoma, kidney cancer, adrenal cancer, renal cell carcinoma, thyroid cancer, blood cancer, skin cancer, brain cancer, cervical cancer, intestinal cancer, liver cancer, colon cancer, gastric cancer, lung cancer, bowel cancer, head and neck cancer, gastrointestinal cancer, multiple myeloma, lymph node cancer, esophageal cancer, colon cancer, rectal cancer, bladder cancer, prostate cancer, endometrial cancer, pancreatic cancer, ear, nose and throat (ENT) cancer, breast cancer, uterine cancer, mammary cancer, prostate cancer, ovarian cancer, and metastases thereof.

Malignant melanoma is a severe type of skin cancer. It is caused by uncontrolled growth of pigment cells called melanocytes.

According to the present disclosure, "epithelial cancer" is a malignant tumor derived from epithelial cells. The group encompasses the most common cancers, including common forms of breast cancer, prostate cancer, lung cancer, and colon cancer.

Lymphomas and leukemias are malignant tumors derived from hematopoietic (blood-forming) cells.

Sarcomas are cancers arising from transformed cells of one of the tissues derived from embryonic mesoderm. Thus, sarcomas include tumors of bone, cartilage, fat, muscle, blood vessels, and hematopoietic tissue.

Blastic tumors or blastomas are tumors (typically malignant) that resemble immature or embryonic tissue. Most of these tumors are commonly found in children.

Glioma is a type of tumor that originates in the brain or spine. It is referred to as glioma because it arises from glial cells. The most common site of glioma is the brain.

### Other components

The pharmaceutical composition of the present disclosure may comprise one or more components in addition to those described in the foregoing sections. For example, the composition may comprise one or more hydrophobic small molecules, such as vitamins (*e*.*g*., vitamin A or vitamin E) or sterols.

The composition may further comprise one or more permeability-enhancing molecules, carbohydrates, polymers, surface-altering agents, or other components. The permeability-enhancing molecules may be, for example, the molecules described in U.S. Patent Application Publication No. 2005/0222064. The carbohydrates may include simple sugars (*e.g.,* glucose) and polysaccharides (*e.g.,* glycogen and derivatives and analogs thereof).

The surface-altering agents may include, but are not limited to, anionic proteins (*e*.*g*., bovine serum albumin), surfactants (*e.g.,* cationic surfactants such as dimethyldioctadecylammonium bromide), sugars or sugar derivatives (*e*.*g*., cyclodextrins), nucleic acids, polymers (*e.g*., heparin, polyethylene glycol, and poloxamer), mucolytic agents (*e.g*., acetylcysteine, Artemisia annua, bromelain, papain, Clerodendrum, bromhexine, carbocisteine, eprazinone, mesna, ambroxol, sobrerol, domiodol, letosteine, stepronin, tiopronin, gelsolin, thymosin β4, dornase alfa, neltenexine, and erdosteine), and DNases (*e.g*., rhDNase). The surface-altering agents may be disposed within and/or on the surface of the nanoparticles of the composition (*e*.*g*., by coating, adsorption, covalent linkage, or other methods).

The composition may further comprise one or more functionalized lipids. For example, lipids may be functionalized with an alkyne group, which may undergo a cycloaddition reaction when exposed to an azide under appropriate reaction conditions. Specifically, lipid bilayers may be functionalized in this manner with one or more groups that are effective in promoting membrane permeation, cell recognition, or imaging. The surface of the composition may also be conjugated with one or more useful antibodies. Functional groups and conjugates suitable for use in targeted cell delivery, imaging, and membrane permeation are well known in the art.

In addition to these components, the composition may comprise any substance suitable for use in pharmaceutical compositions. For example, the composition may include one or more pharmaceutically acceptable (*e*.*g*., pharmacologically acceptable) excipients or auxiliary ingredients, such as, but not limited to, one or more solvents, dispersion media, diluents, dispersion aids, suspension aids, granulation aids, disintegrants, fillers, glidants, liquid vehicles, binders, surfactants, isotonic agents, thickeners or emulsifiers, buffers, lubricants, oils, preservatives, flavoring agents, coloring agents, *etc.*

The term "pharmaceutically acceptable" refers to the non-toxicity of a material, which does not affect the action of the active ingredient of the pharmaceutical composition. Non-pharmaceutically acceptable ingredients may be used to prepare pharmaceutically acceptable ingredients and are included in the present disclosure.

Suitable buffers for use in the compositions of the present disclosure include salt forms of acetic acid, salt forms of citric acid, salt forms of boric acid, and salt forms of phosphoric acid.

When used in the present disclosure, the term "excipient" is intended to mean all substances that may be present in the pharmaceutical compositions of the present disclosure and that are not active ingredients, such as carriers, binders, lubricants, thickeners, surfactants, preservatives, emulsifiers, buffers, flavoring agents, or coloring agents. Excipients are, for example, starch, lactose, or dextrin. Pharmaceutically acceptable excipients are well known in the art (see, *e*.*g*., Remington: The Science and Practice of Pharmacy, 21st Edition, A.R. Gennaro; Lippincott, Williams & Wilkins, Baltimore, MD, 2006).

Suitable preservatives for use in the compositions of the present disclosure include benzalkonium chloride, chlorobutanol, parabens, and thimerosal.

Examples of diluents may include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dry starch, corn starch, powdered sugar, and/or combinations thereof.

### Dosage form and administration

The compositions of the present disclosure may be formulated into preparations in the form of solids, semisolids, liquids, or gases, such as tablets, capsules, ointments, elixirs, syrups, solutions, emulsions, suspensions, injections, and aerosols. The compositions of the present disclosure may be prepared by methods well known in the pharmaceutical art. For example, sterile injectable solutions may be prepared by incorporating the desired amount of a therapeutic or prophylactic agent with various other ingredients described above as required into an appropriate solvent, such as sterile distilled water, followed by filter sterilization. Surfactants may also be added to facilitate the formation of a homogeneous solution or suspension.

For example, the compositions of the present disclosure may be administered intravenously, intramuscularly, intradermally, subcutaneously, intranasally, or by inhalation. In one embodiment, the composition is administered intravenously or subcutaneously.

### Therapeutically effective amount

A "therapeutically effective amount" is an amount of a therapeutic agent that, when administered to a patient, ameliorates a disease or symptom. A "prophylactically effective amount" is an amount of a prophylactic agent that, when administered to a subject, prevents a disease or symptom. The amount of a therapeutic agent constituting a "therapeutically effective amount" or the amount of a prophylactic agent constituting a "prophylactically effective amount" varies depending on the therapeutic and/or prophylactic agent, the disease condition and its severity, the age and weight of a patient and/or subject to be treated and/or prevented, *etc.* Those of ordinary skill in the art can routinely determine the therapeutically effective amount and the prophylactically effective amount based on their knowledge and the present disclosure.

The compositions of the present disclosure are administered in a therapeutically effective amount, which may vary not only with the particular agent selected but also with the route of administration, the nature of the disease being treated, and the age and condition of the patient, and may ultimately be at the discretion of the attending physician or clinician. For example, the therapeutic or prophylactic agent may be administered to a mammal (*e.g.,* a human) at a dose of about 0.0001 mg/kg to about 10 mg/kg.

### Antigen-presenting cells

An antigen-presenting cell (APC) is a cell that presents (*i.e*., displays) an antigen on its surface in the context of a major histocompatibility complex (MHC). The definition includes cases where only one fragment or more fragments of the antigen are presented. T cells can recognize the complex with their T cell receptors (TCRs). Antigen-presenting cells process antigens and present them to T cells.

Professional antigen-presenting cells are highly effective in internalizing antibodies (either by phagocytosis or by receptor-mediated endocytosis) and then displaying antigen fragments bound to class II MHC molecules on their membranes. T cells recognize and interact with the antigen-class II MHC molecule complex on the membrane of antigen-presenting cells. The antigen-presenting cells then produce additional co-stimulatory signals, leading to T cell activation. Expression of co-stimulatory molecules is a typical feature of professional antigen-presenting cells.

The main types of professional antigen-presenting cells are dendritic cells (which have the broadest range of antigen presentation and are likely the most important antigen-presenting cells), macrophages, B cells, and certain activated epithelial cells.

Dendritic cells are a class of leukocytes, including plasmacytoid dendritic cells (pDCs) and conventional dendritic cells (cDCs), which present antigens captured in peripheral tissues to T cells via both class II and class I MHC antigen presentation pathways. Dendritic cells are potent inducers of immune responses, and activation of these cells is a critical step in inducing antitumor immunity.

Antigen-presenting cells can be loaded with MHC-presented peptides by transduction with a nucleic acid encoding a peptide or protein comprising the peptide to be presented (*e.g*., a nucleic acid encoding an antigen (*e.g.,* RNA)). Transfection of dendritic cells with mRNA is a promising antigen-loading technique for stimulating robust antitumor immunity.

The term "immunogenicity" refers to the relative efficiency of an antigen to induce an immune response.

The terms "T cell" and "T lymphocyte" are used interchangeably herein, and include helper T cells (CD4⁺ T cells) and cytolytic or cytotoxic T cells (CTLs, CD8⁺ T cells).

T cells belong to a group of white blood cells known as lymphocytes and play a central role in cell-mediated immunity. They can be distinguished from other types of lymphocytes (*e.g*., B cells and natural killer cells) by the presence of special receptors on their cell surface called T cell receptors (TCRs). The thymus is the primary organ responsible for T cell maturation. Several distinct T cell subsets have been identified, each with different functions.

Helper T cells assist other white blood cells in immune processes, including functions such as maturation of B cells into plasma cells and activation of cytotoxic T cells and macrophages. Since helper T cells express CD4 protein on their surface, these cells are also referred to as CD4⁺ T cells. Helper T cells are activated when class II MHC molecules expressed on the surface of antigen-presenting cells (APCs) present peptide antigens to them. Upon activation, the helper T cells rapidly divide and secrete small proteins called cytokines, which regulate or assist active immune responses.

Cytotoxic T cells destroy diseased cells, such as infected cells (*e*.*g*., virus-infected cells) and cancer cells, and are also involved in transplant rejection. Since cytotoxic T cells express CD8 glycoprotein on their surface, these cells are also referred to as CD8⁺ T cells. These cells recognize their targets by binding to antigens associated with class I MHC molecules, which are present on the surface of nearly every cell in the body.

Most T cells possess a T cell receptor (TCR) that is present as a complex of several proteins. The actual T cell receptor is composed of two separate peptide chains, which are produced by independent T cell receptor α and β (TCRα and TCRβ) genes, and are referred to as α-TCR chain and β-TCR chain. γδ T cells represent a minor subset of T cells that possess a unique T cell receptor (TCR) on their surface. However, in γδ T cells, the TCR is composed of one γ chain and one δ chain. The subset of T cells is less common than αβ T cells (2% of total T cells).

All T cells originate from hematopoietic stem cells in the bone marrow. Hematopoietic progenitor cells derived from hematopoietic stem cells reside in the thymus and proliferate through cell division to generate a large population of immature thymocytes. Early thymocytes express neither CD4 nor CD8 and are thus classified as double-negative (CD4⁻CD8⁻) cells. As the cells progress through development, they become double-positive (CD4⁺CD8⁺) thymocytes and ultimately mature into single-positive (CD4⁺CD8⁻ or CD4⁻CD8⁺) thymocytes before being released from the thymus to peripheral tissues.

The first signal for T cell activation is provided by binding of the T cell receptor to a short peptide presented by the major histocompatibility complex (MHC) on another cell. This ensures that only T cells with TCRs specific to the peptide are activated. Companion cells are typically professional antigen-presenting cells (APCs), typically dendritic cells in the case of primary responses, but B cells and macrophages may also serve as important APCs. Peptides presented by class I MHC molecules to CD8⁺ T cells are 8 to 10 amino acids in length; peptides presented by class II MHC molecules to CD4⁺ T cells are longer, as the binding cleft of class II MHC molecules is open at both ends.

On the basis of common sense in the art, the aforementioned preferred conditions can be combined arbitrarily to obtain preferred examples of the present disclosure.

All reagents and starting materials used herein are commercially available.

The positive and progressive effects of the present disclosure are as follows: compared with the prior art, the mRNA composition prepared by using the compound of formula (I) provided by the present disclosure has one or more of the following advantages:
1. favorable particle size and uniform particle distribution;
2. a significant increase in the protein expression of antigens in the body, muscle, or spleen of subjects (*e.g*., mice);
3. a significant increase in the percentage of antigen-presenting cells expressing antigens in the spleen;
4. a significant enhancement in the maturation status of antigen-presenting cells in the spleen;
5. a significant enhancement in the activation status of T cells in the spleen;
6. a significant decrease in tumor volume and a significant increase in the survival rate of mice.

### BRIEF DESCRIPTION OF THE DRAWINGS

To more clearly illustrate the technical solutions of the embodiments of the present disclosure, the accompanying drawings of the present disclosure will be briefly described below. It should be understood that the accompanying drawings in the following description relate only to some specific embodiments of the present disclosure and are not intended to limit the present disclosure.
FIG. 1 shows fluorescence images of *in vivo* mice and mouse organs (liver and spleen) 6 hours after intravenous injection of a Fluc-mRNA-TLP composition prepared with YK-1202, YK-1204, YK-1205, YK-1206, or YK-1208, and a Fluc-mRNA-TLP composition without adjuvant lipid. (Left: *in vivo* mice; Right: mouse organs)
FIG. 2 shows flow cytometry results (eGFP) of spleen cells from mice 24 hours after intravenous injection of a blank, an eGFP-mRNA-TLP composition supplemented with YK-1202 or YK-1204, and an eGFP-mRNA-TLP composition without adjuvant lipid.
FIG. 3 shows flow cytometry results (CD86) of spleen cells from mice 24 hours after intravenous injection of a blank or an eGFP-mRNA-TLP composition supplemented with YK-1205 or TMX-201.
FIG. 4 shows flow cytometry results (CD69) of spleen cells from mice 24 hours after intravenous injection of a blank or a TLP composition supplemented with YK-1204, YK-1205, or TMX-201.
FIG. 5 shows the induction of cytokines IFN-γ and IL-12 in serum of B16F10-OVA mice immunized with an OVA mRNA-TLP composition supplemented with YK-1202, YK-1204, YK-1205, or YK-1208.
FIG. 6 shows the tumor growth in B16F10-OVA mice immunized with an OVA mRNA-TLP composition supplemented with YK-1202, YK-1204, YK-1205, or YK-1208.
FIG. 7 shows the survival rate of B16F10-OVA mice immunized with an OVA mRNA-TLP composition supplemented with YK-1202, YK-1204, YK-1205, or YK-1208.
FIG. 8 shows the induction of cytokines IFN-γ and IL-12 in serum of B16F10-OVA mice immunized with an OVA mRNA-LNP composition supplemented with YK-1202, YK-1204, YK-1206, or YK-1208.
FIG. 9 shows the tumor growth in MC38-OVA mice immunized with an OVA mRNA-LNP composition supplemented with YK-1202, YK-1204, YK-1206, or YK-1208.
FIG. 10 shows the survival rate of MC38-OVA mice immunized with an OVA mRNA-LNP composition supplemented with YK-1202, YK-1204, YK-1206, or YK-1208.

### DETAILED DESCRIPTION OF THE PREFERRED EMBODIMENT

The present disclosure is further described below by way of examples. However, the present disclosure is not limited to the scope of the following examples.

The implementation conditions used in the examples may be further adjusted according to different requirements of specific use, and the implementation conditions not specified are conventional in the art. In the specific examples of the present disclosure, all starting materials used are commercially available. Unless otherwise specified, all percentages in the context are by weight, and all temperatures are given in degrees Celsius. The technical features involved in various embodiments of the present disclosure may be combined as long as they do not conflict with each other.

The following abbreviations represent the following reagents:
ADOPE: sodium 2-acetamidoethyl ((R)-2,3-bis(oleoyloxy)propyl) phosphate; ( 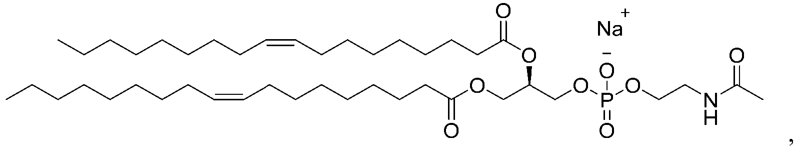 prepared according to Example 1 of Patent CN118001254A)
DOTMA: 1,2-di-O-octadecenyl-3-trimethylammonium propane (chloride salt);
DOPE: 1,2-dioleoyl-sn-glycero-3-phosphoethanolamine;
DOPG: 1,2-dioleoyl-sn-glycero-3-phospho-rac-(1-glycerol) sodium salt;
DSPC: 1,2-distearoyl-sn-glycero-3-phosphocholine;
DMG-PEG2000: 1,2-dimyristoyl-sn-glycero-3-methoxypolyethylene glycol 2000;
Boc₂O: di-*tert*-butyl dicarbonate;
DMAP: 4-dimethylaminopyridine;
EDCI: 1-ethyl-(3-dimethylaminopropyl)carbodiimide hydrochloride;
TsOH: *p*-toluenesulfonic acid;
TFA: trifluoroacetic acid;
DCM: dichloromethane;
DMF: *N*,*N*-dimethylformamide;
THF: tetrahydrofuran;
TrCl: triphenylmethyl chloride.

### Example 1: Synthesis of conjugated lipid-based TLR adjuvant

### 1. Synthesis of YK-1201

The synthetic route of YK-1201 was as follows:

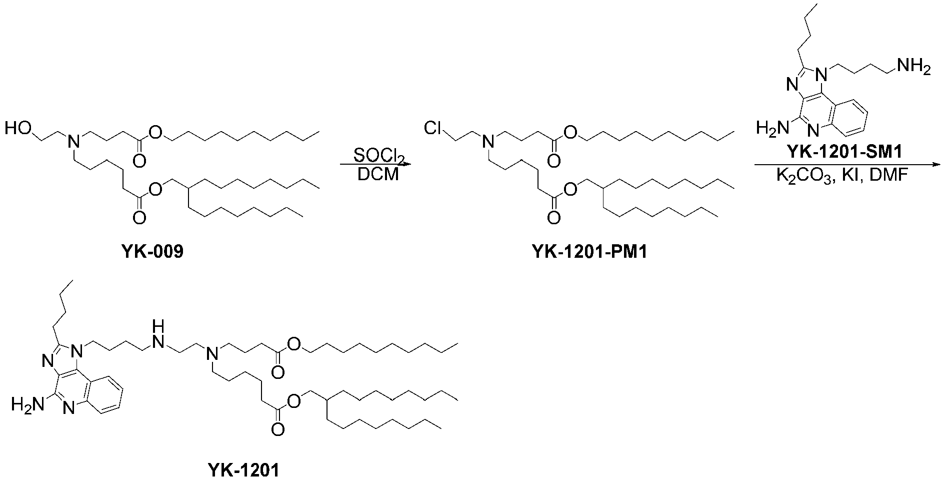

### Step 1: Synthesis of YK-1201-PM1

YK-009 (500 mg, 0.765 mmol) was dissolved in dichloromethane (10 mL), followed by dropwise addition of thionyl chloride (180 mg, 1.530 mmol) in an ice-water bath, and the mixture was stirred at room temperature for 6 hours. After the reaction was completed, the solvent was evaporated to dryness by rotary evaporation, and the residue was purified by silica gel chromatography (0% to 15% dichloromethane/methanol) to obtain the product YK-1201-PM1 (500 mg, 0.743 mmol, 97.1%).

### Step 2: Synthesis of YK-1201

YK-1201-PM1 (150 mg, 0.223 mmol) and YK-1201-SM1 (84 mg, 0.270 mmol) were dissolved in DMF (3 mL), followed by addition of potassium carbonate (93 mg, 0.673 mmol) and potassium iodide (3 mg, 0.018 mmol) at room temperature, and the mixture was stirred at 70°C for 6 hours. After the reaction was completed, the reaction mixture was cooled, followed by sequential addition of ethyl acetate and water. The phases were separated, and the aqueous phase was back-extracted twice with ethyl acetate. The organic phases were combined, and the solvent was evaporated to dryness by rotary evaporation to obtain a crude product, which was purified by silica gel chromatography (0% to 15% dichloromethane/methanol) to obtain the product YK-1201 (120 mg, 0.127 mmol, 57.0%). C₅₈H₁₀₂N₆O₄, MS (ES): m/z (1/2 M + Na⁺) = 496.63.

YK-1201: ¹H NMR (400 MHz, DMSO-*d*₆) δ 7.98 (d, *J* = 8.0 Hz, 1H), 7.60 (d, *J* = 8.0 Hz, 1H), 7.43 - 7.35 (m, 1H), 7.26 - 7.19 (m, 1H), 6.42 (s, 2H), 4.52 - 4.44 (m, 2H), 3.99 - 3.86 (m, 6H), 3.07 - 2.96 (m, 2H), 2.94 - 2.86 (m, 2H), 2.40 - 2.30 (m, 4H), 2.29 - 2.21 (m, 4H), 1.85 - 1.74 (m, 4H), 1.62 - 1.40 (m, 12H), 1.29 - 1.17 (m, 45H), 1.13 - 1.05 (m, 2H), 0.96 (t, *J* = 7.2 Hz, 3H), 0.88 - 0.79 (m, 9H).

### 2. Synthesis of YK-1202

The synthetic route of YK-1202 was as follows:

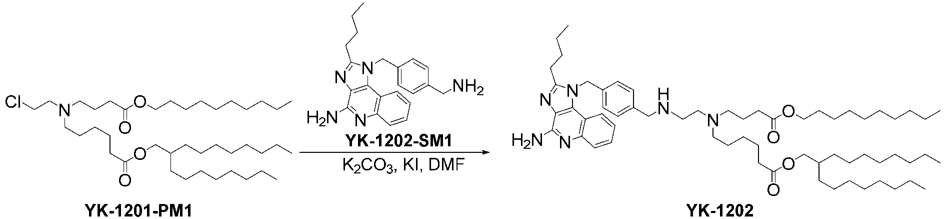

According to the synthesis method of YK-1201, YK-1201-PM1 (100 mg, 0.149 mmol) and YK-1202-SM1 (64 mg, 0.178 mmol) were used as starting materials to obtain the product YK-1202 (34 mg, 0.0342 mmol, 22.9%). C₆₂H₁₀₂N₆O₄, MS (ES): m/z (1/2 M +Na⁺) = 520.58.

YK-1202: ¹H NMR (400 MHz, DMSO-*d*₆) δ 7.79 (d, *J* = 8.0 Hz, 1H), 7.59 (d, *J* = 8.0 Hz, 1H), 7.38 - 7.33 (m, 1H), 7.25 - 7.20 (m, 2H), 7.10 - 7.00 (m, 3H), 6.82 - 6.67 (m, 2H), 5.86 (s, 2H), 4.36 - 4.29 (m, 3H), 4.15 - 3.86 (m, 3H), 3.19 - 3.15 (m, 2H), 2.94 - 2.65 (m, 5H), 2.41 - 2.14 (m, 4H), 2.03 - 1.89 (m, 2H), 1.75 - 1.65 (m, 4H), 1.63 - 0.90 (m, 54H), 0.89 - 0.79 (m, 12H).

### 3. Synthesis of YK-1203

The synthetic route of YK-1203 was as follows:

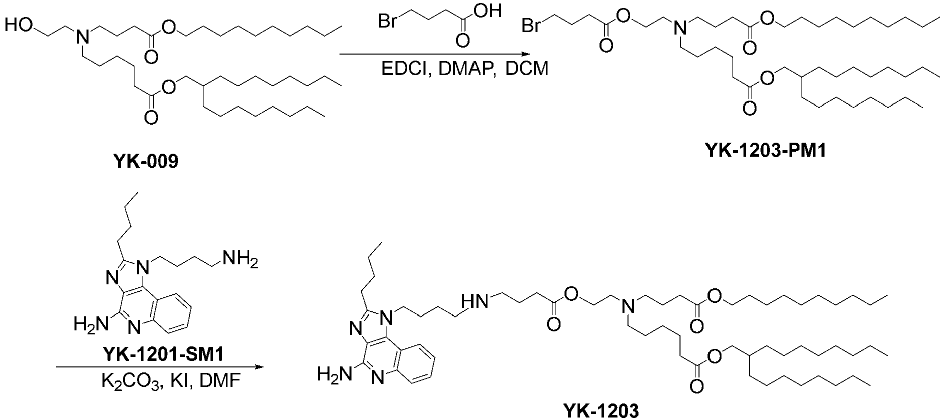

### Step 1: Synthesis of YK-1203-PM1

YK-009 (500 mg, 0.764 mmol) and 4-bromobutyric acid (612 mg, 3.665 mmol) were dissolved in dichloromethane (5 mL), followed by addition of EDCI (440 mg, 2.295 mmol) and DMAP (38 mg, 0.311 mmol) at room temperature, and the mixture was stirred at 35°C for 16 hours. After the reaction was completed, the solvent was evaporated to dryness by rotary evaporation to obtain a crude product, which was purified by silica gel chromatography (0% to 15% dichloromethane/methanol) to obtain the product YK-1203-PM1 (480 mg, 0.598 mmol, 78.2%).

### Step 2: Synthesis of YK-1203

According to the synthesis method of YK-1201, YK-1203-PM1 (150 mg, 0.187 mmol) and YK-1201-SM1 (70 mg, 0.224 mmol) were used as starting materials to obtain the product YK-1203 (24 mg, 0.0232 mmol, 12.4%). C₆₂H₁₀₈N₆O₆, MS (ES): m/z (1/2 M +Na⁺) = 539.68.

YK-1203: ¹H NMR (400 MHz, DMSO-*d*₆) δ 8.01 (d, *J* = 8.0 Hz, 1H), 7.64 (d, *J* = 8.0 Hz, 1H), 7.48 - 7.40 (m, 1H), 7.31 - 7.24 (m, 1H), 6.94 - 6.56 (m, 2H), 4.55 - 4.45 (m, 2H), 4.02 - 3.87 (m, 6H), 3.07 - 2.96 (m, 2H), 2.94 - 2.86 (m, 2H), 2.63 - 2.54 (m, 2H), 2.40 - 2.30 (m, 4H), 2.29 - 2.21 (m, 4H), 1.85 - 1.70 (m, 6H), 1.62 - 1.40 (m, 10H), 1.29 - 1.17 (m, 50H), 1.13 - 1.05 (m, 2H), 0.96 (t, *J* = 7.2 Hz, 3H), 0.88 - 0.79 (m, 9H).

### 4. Synthesis of YK-1204

The synthetic route of YK-1204 was as follows:

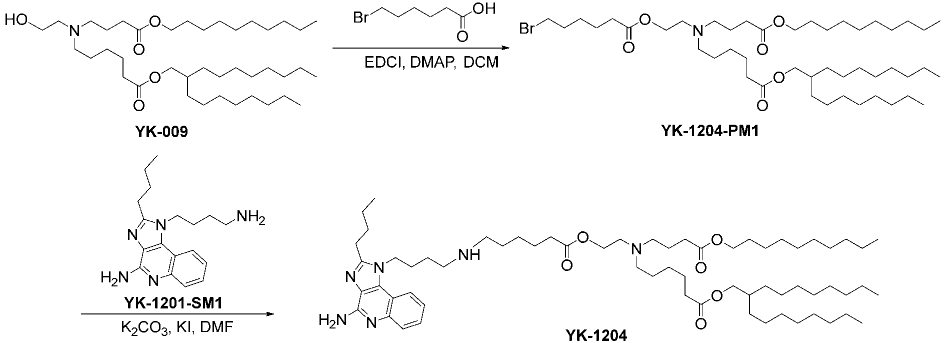

### Step 1: Synthesis of YK-1204-PM1

According to the synthesis method of YK-1203-PM1, YK-009 (500 mg, 0.764 mmol) and 6-bromohexanoic acid (596 mg, 3.058 mmol) were used as starting materials to obtain the product YK-1204-PM1 (520 mg, 0.626 mmol, 81.9%).

### Step 2: Synthesis of YK-1204

According to the synthesis method of YK-1201, YK-1204-PM1 (150 mg, 0.180 mmol) and YK-1201-SM1 (84 mg, 0.271 mmol) were used as starting materials to obtain the product YK-1204 (60 mg, 0.0565 mmol, 31.4%). C₆₄H₁₁₂N₆O₆, MS (ES): m/z (1/2 M + Na⁺) = 553.57.

YK-1204: ¹H NMR (400 MHz, DMSO-*d*₆) δ 8.04 (d, *J* = 8.0 Hz, 1H), 7.60 (d, *J* = 8.0 Hz, 1H), 7.45 - 7.36 (m, 1H), 7.26 - 7.20 (m, 1H), 6.42 (s, 2H), 4.57 - 4.47 (m, 2H), 4.03 - 3.93 (m, 4H), 3.92 - 3.86 (m, 2H), 2.95 - 2.87 (m, 2H), 2.72 - 2.53 (m, 4H), 2.41 - 2.31 (m, 4H), 2.30 - 2.20 (m, 6H), 1.89 - 1.75 (m, 4H), 1.63 - 1.39 (m, 14H), 1.29 - 1.17 (m, 50H), 1.13 - 1.05 (m, 2H), 0.96 (t, *J* = 7.2 Hz, 3H), 0.87 - 0.80 (m, 9H).

### 5. Synthesis of YK-1205

The synthetic route of YK-1205 was as follows:

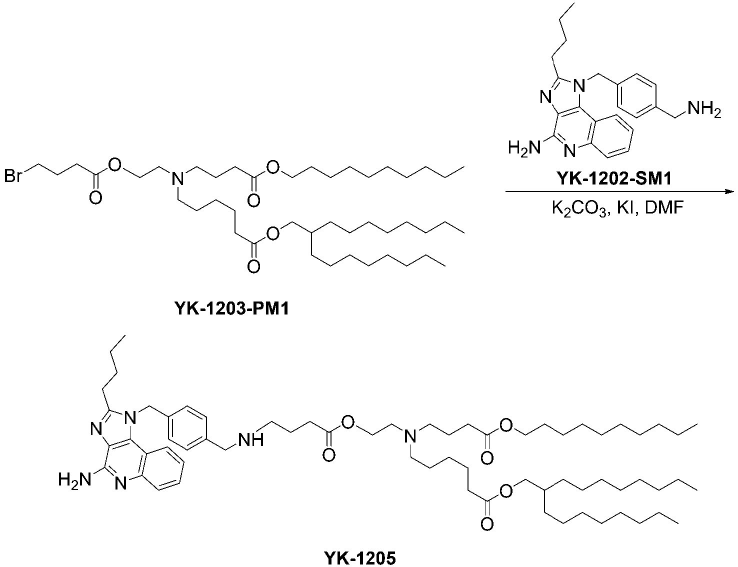

According to the synthesis method ofYK-1201, YK-1203-PM1 (100 mg, 0.125 mmol) and YK-1202-SM1 (66 mg, 0.184 mmol) were used as starting materials to obtain the product YK-1205 (60 mg, 0.0555 mmol, 44.4%). C₆₆H₁₀₈N₆O₆, MS (ES): m/z (1/2 M + Na⁺) = 563.66.

YK-1205: ¹H NMR (400 MHz, DMSO-*d*₆) δ 7.80 (d, *J* = 8.0 Hz, 1H), 7.60 (d, *J* = 8.0 Hz, 1H), 7.40 - 7.33 (m, 1H), 7.18 (d, *J* = 8.4 Hz, 2H), 7.11 - 7.04 (m, 1H), 7.18 (d, *J* = 8.4 Hz, 2H), 5.84 (s, 2H), 4.13 - 4.08 (m, 2H), 4.01 - 3.89 (m, 8H), 2.93 - 2.86 (m, 2H), 2.38 - 2.32 (m, 4H), 2.30 - 2.24 (m, 4H), 1.78 - 1.68 (m, 4H), 1.59 - 1.47 (m, 10H), 1.41 - 1.30 (m, 8H), 1.29 - 1.23 (m, 42H), 0.89 - 0.80 (m, 12H).

### 6. Synthesis of YK-1206

The synthetic route of YK-1206 was as follows:

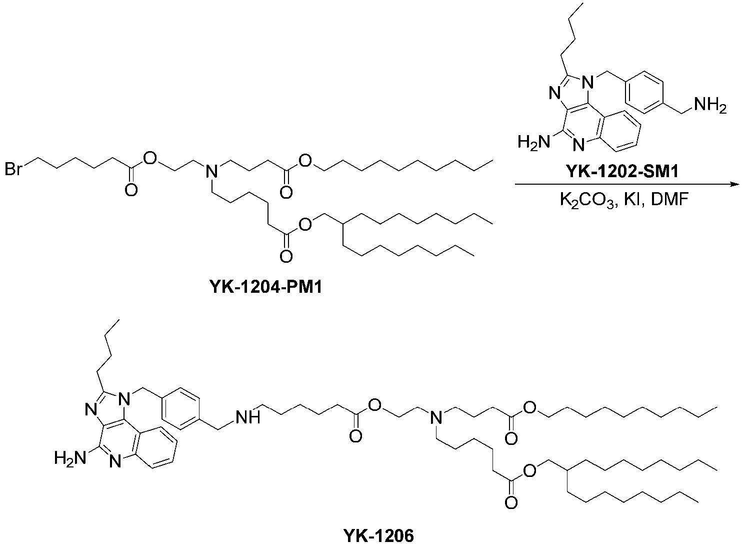

According to the synthesis method of YK-1201, YK-1204-PM1 (100 mg, 0.120 mmol) and YK-1202-SM1 (52 mg, 0.144 mmol) were used as starting materials to obtain the product YK-1205 (40 mg, 0.0360 mmol, 30.0%). C₆₈H₁₁₂N₆O₆, MS (ES): m/z (1/2 M + Na⁺) = 577.72.

YK-1206: ¹H NMR (400 MHz, DMSO-*d*₆) δ 7.80 (d, *J=* 8.0 Hz, 1H), 7.60 (d, *J* = 8.0 Hz, 1H), 7.40 - 7.33 (m, 1H), 7.18 (d, *J* = 8.4 Hz, 2H), 7.11 - 7.04 (m, 1H), 7.18 (d, *J* = 8.4 Hz, 2H), 5.84 (s, 2H), 4.01 - 3.96 (m, 4H), 3.95 - 3.86 (m, 6H), 2.93 - 2.87 (m, 2H), 2.40 - 2.34 (m, 4H), 2.27 - 2.24 (m, 4H), 1.76 - 1.63 (m, 4H), 1.56 - 1.45 (m, 14H), 1.38 - 1.30 (m, 8H), 1.27 - 1.23 (m, 42H), 0.88 - 0.81 (m, 12H).

### 7. Synthesis of YK-1207

The synthetic route of YK-1207 was as follows:

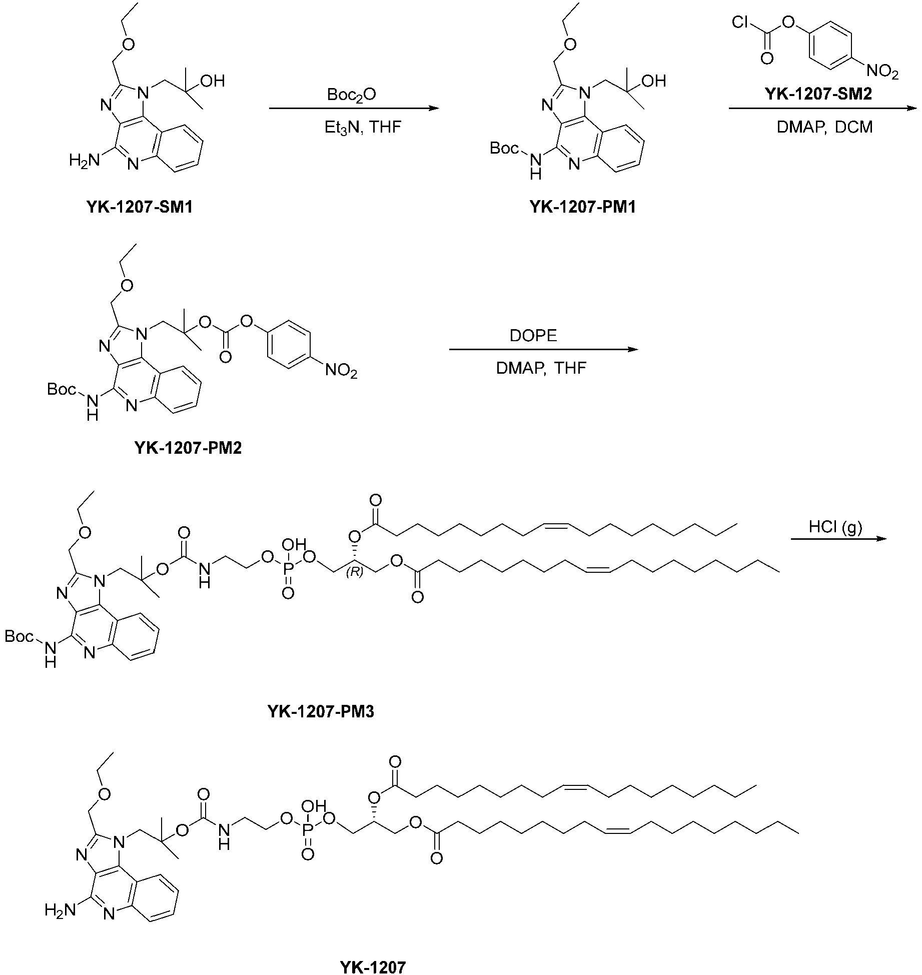

### Step 1: Synthesis of YK-1207-PM1

YK-1207-SM1 (830 mg, 2.64 mmol) was dissolved in tetrahydrofuran (10 mL). The mixture was cooled to 0°C, and triethylamine (1.33 g, 13.15 mmol) was slowly added dropwise thereto. After stirring for 5 minutes, Boc₂O (2.88 g, 13.20 mmol) was added dropwise thereto. The reaction mixture was allowed to warm to room temperature and stirred overnight. The reaction was monitored by LCMS. After the reaction was completed, water and ethyl acetate were sequentially added to the reaction mixture. The phases were separated, and the aqueous phase was back-extracted twice with ethyl acetate. The organic phases were combined, and the solvent was evaporated to dryness by rotary evaporation to obtain a crude product, which was purified by silica gel chromatography (0% to 10% dichloromethane/methanol) to obtain the product YK-1207-PM1 (830 mg, 2.00 mmol, 75.8%). C₂₂H₃₀N₄O₄, MS (ES): m/z (M + H⁺) = 415.38.

### Step 2: Synthesis of YK-1207-PM2

YK-1207-PM1 (300 mg, 0.72 mmol) was dissolved in dichloromethane (3.5 mL). The mixture was cooled to 0°C, and DMAP (531 mg, 4.30 mmol) was added thereto. After stirring for 5 minutes, YK-1207-SM2 (437 mg, 2.16 mmol) was added thereto. The reaction mixture was allowed to warm to room temperature and stirred for 4 hours. The reaction was monitored by TLC. After the reaction was completed, the residue was purified by silica gel chromatography (0% to 50% dichloromethane/ethyl acetate) to obtain the product YK-1207-PM2 (105 mg, 0.181 mmol, 25.2%). C₂₉H₃₃N₅O₈, MS (ES): m/z (M + H⁺) = 580.75.

### Step 3: Synthesis of YK-1207-PM3

YK-1207-PM2 (105 mg, 0.18 mmol) was dissolved in dichloromethane (3 mL), followed by addition of DIEA (94 mg, 0.73 mmol) and DOPE (539 mg, 0.73 mmol) at room temperature, and the mixture was stirred overnight at room temperature. The reaction was monitored by TLC. After the reaction was completed, the solvent was evaporated to dryness by rotary evaporation to obtain a crude product, which was purified by silica gel chromatography (0% to 10% dichloromethane/methanol) to obtain the product YK-1207-PM3 (100 mg, 0.084 mmol, 46.9%). C₆₄H₁₀₆N₅O₁₃P, MS (ES): m/z (M + H⁺) = 1185.17.

### Step 4: Synthesis of YK-1207

YK-1207-PM3 (100 mg, 0.084 mmol) was dissolved in a solution of hydrogen chloride in ethyl acetate (4 M, 1 mL), and the mixture was stirred at room temperature for 4 hours. The reaction was monitored by LCMS. After the reaction was completed, the solvent was evaporated to dryness by rotary evaporation to obtain a crude product, which was purified by silica gel chromatography (0% to 10% dichloromethane/methanol) to obtain the product YK-1207 (20 mg, 0.018 mmol, 21.9%). C₅₉H₉₈N₅O₁₁P, MS (ES): m/z (M + H⁺) = 1085.13.

YK-1207: ¹H NMR (400 MHz, DMSO-*d*₆) δ 8.44 (d, *J* = 8.0 Hz, 1H), 8.10 (d, *J* = 8.0 Hz, 1H), 7.55 (d, *J* = 7.6 Hz, 1H), 7.46 (d, *J* = 7.6 Hz, 1H), 5.34 - 5.24 (m, 4H), 5.07 - 4.99 (m, 1H), 4.78 - 4.68 (m, 1H), 4.25 - 4.18 (m, 1H), 3.99 - 3.94 (m, 1H), 3.87 - 3.76 (m, 4H), 3.56 - 3.48 (m, 6H), 2.95 - 2.93 (m, 10H), 2.16 - 2.12 (m, 2H), 2.04 - 1.92 (m, 8H), 1.44 - 1.32 (m, 9H), 1.27 - 1.21 (m, 48H), 0.88 - 0.81 (m, 9H).

### 8. Synthesis of YK-1208

The synthetic route of YK-1208 was as follows:

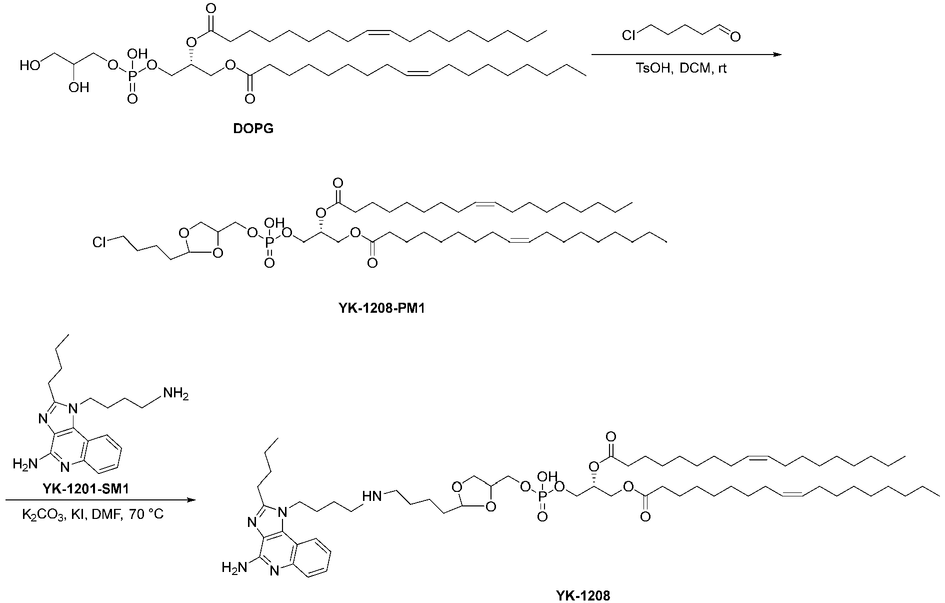

### Step 1: Synthesis of YK-1208-PM1

5-Chloropentanal (77 mg, 0.639 mmol) was dissolved in dichloromethane (10 mL), followed by addition of DOPG (500 mg, 0.645 mmol) and *p*-toluenesulfonic acid (62 mg, 0.33 mmol) at room temperature, and the mixture was stirred overnight at room temperature. The reaction was monitored by TLC. After complete consumption of the starting materials and completion of the reaction, the reaction mixture was poured into saturated sodium bicarbonate aqueous solution and extracted with dichloromethane. The organic phases were combined, and the solvent was evaporated to dryness by rotary evaporation to obtain a crude product, which was purified by silica gel chromatography (0% to 10% dichloromethane/methanol) to obtain the product YK-1208-PM1 (420 mg, 0.479 mmol, 74.9%). C₄₇H₈₆ClO₁₀P, MS (ES): m/z (M - H⁺) = 875.84.

### Step 2: Synthesis of YK-1208

YK-1208-PM1 (320 mg, 0.364 mmol) and YK-1201-SM1 (114 mg, 0.366 mmol) were dissolved in DMF (3.5 mL), followed by addition of potassium carbonate (150 mg, 1.10 mmol) and potassium iodide (6 mg, 0.040 mmol) at room temperature, and the mixture was stirred at 70°C for 16 hours. After completion of the reaction, the reaction mixture was cooled, poured into saturated sodium bicarbonate aqueous solution, and extracted with ethyl acetate. The organic phases were combined, and the solvent was evaporated to dryness by rotary evaporation to obtain a crude product, which was purified by silica gel chromatography (0% to 20% dichloromethane/methanol) to obtain the product YK-1208 (105 mg, 0.091 mmol, 24.6%). C₆₅H₁₁₀N₅O₁₀P, MS (ES): m/z (M + H⁺) = 1153.26.

¹H NMR (400 MHz, CDCl₃) δ 7.94 - 8.00 (m, 2H), 7.48 - 7.54 (m, 2H), 5.27 - 5.33 (m, 8H), 4.88 - 5.03 (m, 2H), 4.52 (s, 1H), 4.41 - 4.44 (m, 2H), 4.20 (s, 3H), 4.00 - 4.08 (m, 6H), 3.49 (t, *J =* 8.0, 2H), 2.91 (t, *J =* 8.0, 4H), 2.24 - 2.32 (m, 8H), 2.00 (s, 16H), 1.85 - 1.88 (m, 6H), 1.76 - 1.79 (m, 5H), 1.58 (s, 8H), 1.27 (s, 23H), 1.00 (t, *J =* 8.0, 3H), 0.86 - 0.88 (m, 9H).

### 9. Synthesis of TMX 201

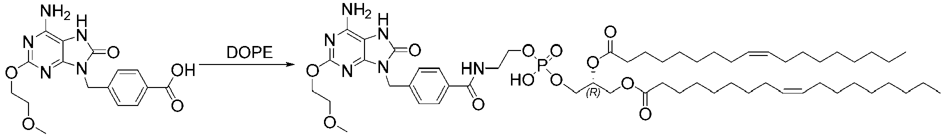

TMX 201 was synthesized with reference to the synthetic route of Compound A in WO2011134669A, yielding 25 mg of TMX 201.

### 10. Synthesis of Compound 23

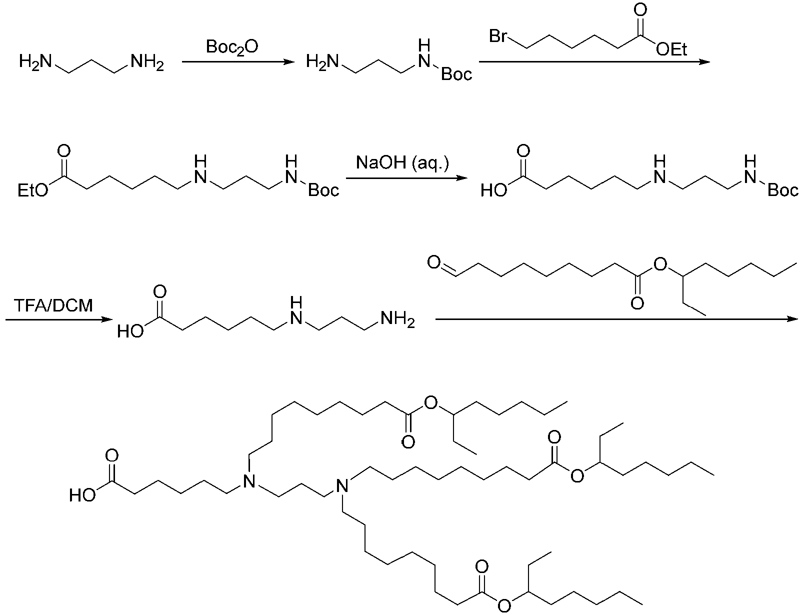

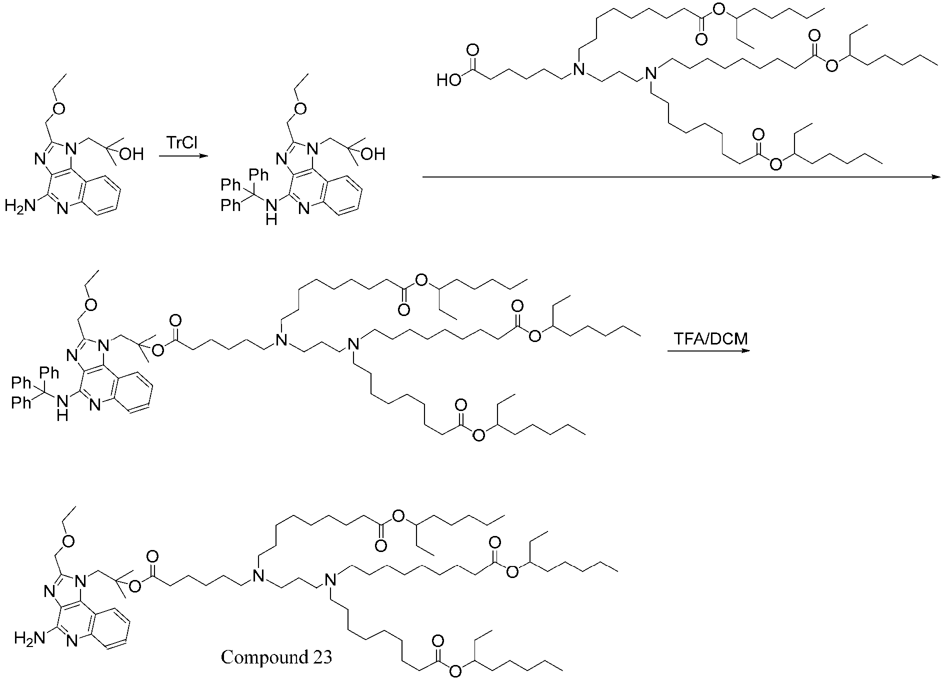

Compound 23 was synthesized with reference to the synthetic route of Compound 23 in WO2021237055A1, yielding 30 mg of Compound 23.

### 11. Synthesis of 63-15

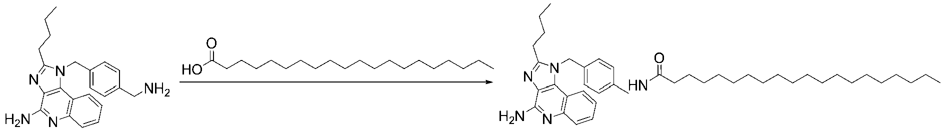

63-15 was synthesized with reference to the synthetic route of Compound No. 63-15 in WO2019040491A1, yielding 42 mg of 63-15.

### Example 2: Preparation of mRNA lipid composition

### A) Preparation of Fluc DNA, eGFP DNA, and OVA DNA templates

1) Circular plasmids encoding luciferase (Luciferase CDS), green fluorescent protein (GFP), and ovalbumin (OVA) were digested with EcoRV and ligated into a pVAX1 vector (purchased from Thermo Fisher Scientific).
2) The constructed plasmid from step 1) on the pVAX1 vector was mixed with 50 µL of *E. coli* competent cells Stbl2 (purchased from Thermo Fisher Scientific) and incubated on ice for 30 minutes, followed by heat shock at 42°C for 90 seconds, immediately returned to ice, and incubated on ice for 2 minutes.
3) 400 µL of LB medium (purchased from Thermo Fisher Scientific) was added, and the mixture was incubated with gentle oscillation on an orbital shaker at 30°C for 45 to 60 minutes.
4) 50 to 100 µL of bacterial suspension was spread on LB solid medium containing kanamycin antibiotic (100 µg/mL, purchased from Yeasen Biotechnology Co., Ltd.) for inverted culture at 37°C overnight.
5) The resulting monoclonal colonies on the plate were subjected to sequencing verification for correctness, and correctly sequenced monoclonal colonies were picked and incubated with gentle oscillation on an orbital shaker at 30°C overnight.
6) Plasmid extraction was performed using an endotoxin-free plasmid maxi-prep kit (purchased from Yeasen Biotechnology Co., Ltd.).
7) The extracted plasmid was digested with restriction endonucleases to form a linearized plasmid for use as a transcription template. The specific enzymatic digestion procedure is detailed in steps (i) to (iii).

Step (i): 1 mg of luciferase circular plasmid was digested with BspQ I enzyme (purchased from Yeasen Biotechnology Co., Ltd.) at 37°C for 4 hours to produce a linearized DNA transcription template (see Table 1 for the enzymatic digestion system).

**Table 1: Enzymatic digestion system**

| **Reaction component** | **Final concentration/volume added** |
|---|---|
| 10× digestion buffer (mL) | 2 mL |
| BspQ I enzyme | 1 KU/mg |
| Luciferase circular plasmid | 50 µg/mL |
| Water for injection | Supplemented to 20 mL |

Step (ii): After the reaction was completed, anhydrous ethanol and 3M sodium acetate were sequentially added at a volume ratio of V_{digestion product}: V_{anhydrous ethanol}: V_{3M sodium acetate} = 1:3:1, followed by precipitation at -20°C for 1 hour. The precipitate was then retained by centrifugation at 12,000 rpm.

Step (iii): The precipitate from step (ii) was washed twice with 70% ethanol. The centrifuged material was dried at 55°C for 10 minutes, followed by dissolution in 1.7 mL of water for injection.

The concentration of the linearized plasmid in the dissolution solution was 500 ng/µL, with a linearization ratio of 90% or more and a purification recovery efficiency of 85%.

### B) Preparation of Fluc mRNA, eGFP mRNA, and OVA mRNA

### 1) Co-transcriptional capping reaction:

Using the Fluc DNA, eGFP DNA, and OVA DNA prepared in A) as templates, and NTP solution (NTPs) and Cap1 analog (Cat. No.: 10678ES80, purchased from Yeasen Biotechnology Co., Ltd.) as starting materials, mRNA was synthesized via transcription using T7 RNA polymerase. The specific reaction system is shown in Table 2. The prepared reaction system was incubated in a 37°C incubator with shaking for 3 hours. The Cap1 analog used was Cap1-GAG, having a structure of m7G (5') ppp (5') (2'-OMeA) pG, with a molecular formula of C₃₂H₄₃N₁₅O₂₄P₄.

**Table 2: Co-transcriptional capping reaction system**

| **Reaction component** | | **Final concentration/volume added** |
|---|---|---|
| T7 transcription buffer (10×) | | T7 transcription buffer: 1/10 of the total volume, with a magnesium ion concentration of 40 mM |
| NTPs | ATP | 10 mM |
| | CTP | 10 mM |
| | GTP | 10 mM |
| | *N1*-Methylpseudo-UTP | 10 mM |
| Cap1 | | 10 mM |
| linearized plasmid | | 50 µg/mL |
| Murine RNase inhibitor | | 1 KU/mL |
| Inorganic pyrophosphatase | | 20 to 40 U per 1 mg of linearized plasmid |
| T7 RNA polymerase | | 150 to 300 KU per 1 mg of linearized plasmid |
| Water for injection | | Supplemented to target volume |

| | | |
|---|---|---|
| Note: All the above reagents were purchased from Yeasen Biotechnology Co., Ltd. | | |

### 2) Digestion of template DNA:

DNase I (purchased from Yeasen Biotechnology Co., Ltd.) was added to the co-transcriptional capping reaction system completed in step 1) above to achieve a final concentration of 1 U/µg linearized plasmid. After mixing and centrifugation, the mixture was incubated at 37°C for 1 hour to obtain a co-transcriptional capping product.

### 3) Purification by lithium chloride precipitation:

The co-transcriptional capping product obtained in step 2) above was purified by lithium chloride precipitation as follows:

Step (i): Addition of lithium chloride: Lithium chloride solution (purchased from Thermo Fisher Scientific) was added to the product obtained in step 2) above to achieve a final concentration of 2.8 M, followed by low-temperature precipitation for 2 hours.

Step (ii): Precipitation: The mixture was centrifuged at 12,000 rpm for 15 minutes, and the precipitate was retained.

Step (iii): Washing: The precipitate was washed twice with 75% ethanol and dissolved in water for injection to obtain an mRNA solution. The purified mRNA solution was stored at -80°C.

### Example 3: Effect of different amounts of adjuvant lipids on TLP-mRNA composition

ADOPE, DOTMA, DOPE, and YK-1202 were weighed according to the molar ratios in Table 3 and dissolved in ethanol to prepare an ethanol-lipid complex solution (with a total lipid concentration of 266 mM). The ethanol-lipid complex solution was rapidly added to sterile nuclease-free water under stirring at 120 rpm by ethanol injection method, and stirred at room temperature for 30 minutes. The resulting mixture was filtered through a polycarbonate membrane with a pore size of 450 nm to obtain a lipid complex solution, which was stored at 4°C to 8°C. The mRNA was diluted in a buffer to obtain an mRNA aqueous solution (with an mRNA concentration of 0.5 mg/mL, the buffer being 10 mM HEPES buffer containing 0.1 mM EDTA). A sodium chloride aqueous solution (0.9% w/w) was drawn with a syringe and injected into the prepared mRNA aqueous solution to obtain a mixture of mRNA and sodium chloride. The lipid complex solution was drawn with a syringe and injected into the mixture of mRNA and sodium chloride, vortexed for 30 seconds, and incubated at room temperature for 10 minutes to obtain a TLP pharmaceutical composition with or without adjuvant lipid (with a final RNA concentration of 100 µg/mL), which was stored at 4°C to 8°C. The mRNA and lipid complex solution were used in an amount such that the charge ratio of the TLP-mRNA composition was 1:2. The charge ratio was calculated as follows:

Charge ratio of mRNA-TLP composition = (molar amount of positive charge from permanent cationic lipids - molar amount of negative charge from permanent anionic lipids) / (weight of mRNA (g) divided by average molecular weight per base (330 g/mol)).

The mRNA-TLP composition was diluted by mixing with a sodium chloride aqueous solution (0.9% w/w) at a volume ratio of 1:5. The particle size and polydispersity index (PDI) were determined by dynamic light scattering using a Malvern laser particle size analyzer.

**Table 3: Particle size and PDI of compositions with different formulations**

| No. | ADOPE | DOTMA | DOPE | YK-1202 | Particle size/nm | PDI |
|---|---|---|---|---|---|---|
| 1 | 25 mol% | 47.5 mol% | 25 mol% | 2.5 mol% | 265.0 | 0.1985 |
| 2 | 25 mol% | 45 mol% | 25 mol% | 5 mol% | 290.2 | 0.2746 |
| 3 | 25 mol% | 40 mol% | 25 mol% | 10 mol% | 305.4 | 0.1889 |
| 4 | 25 mol% | 35 mol% | 25 mol% | 15 mol% | 464.2 | 0.4561 |
| 5 | 25 mol% | 30 mol% | 25 mol% | 20 mol% | 480.5 | 0.4863 |

The results demonstrated that when YK-1202 was used to partially replace the permanent cationic lipid DOTMA in the TLP composition at 2.5%, 5.0%, 10.0%, 15.0%, and 20.0%, the particle size and PDI of the resulting TLP pharmaceutical composition were within the qualified range (a particle size of 220 to 500 nm and a PDI of less than 0.5). A 10% adjuvant lipid ratio was used in the following examples.

### Example 4: Effect of different adjuvant lipids on TLP-mRNA composition

**Table 4: Structures of adjuvant compounds used in anionic lipid compositions**

| Name | Structure |
|---|---|
| YK-1201 | 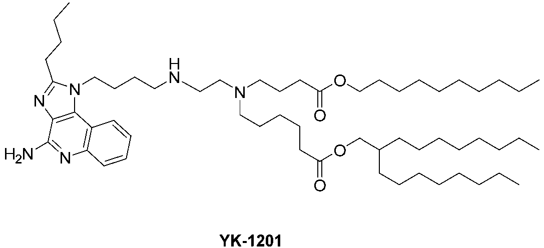 |
| YK-1202 | 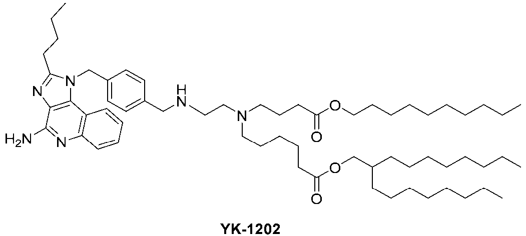 |
| YK-1203 | 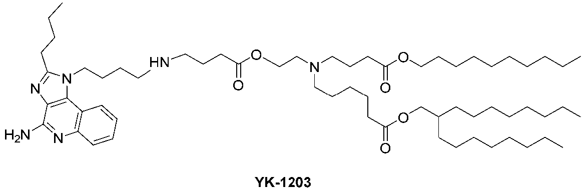 |
| YK-1204 | 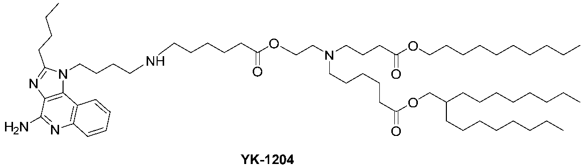 |
| YK-1205 | 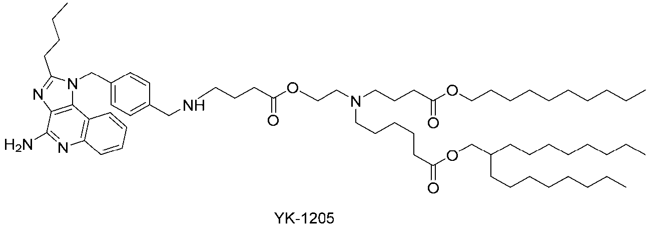 |
| YK-1206 | 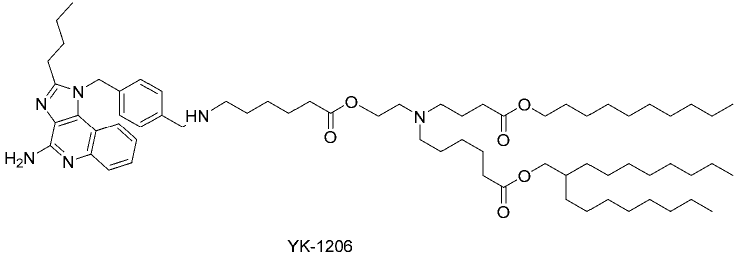 |
| YK-1207 | 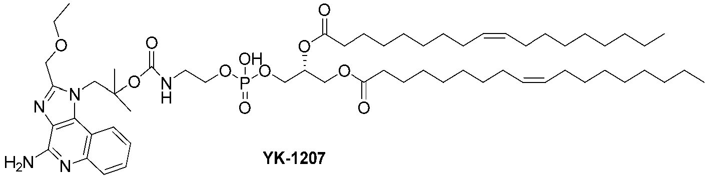 |
| YK-1208 | 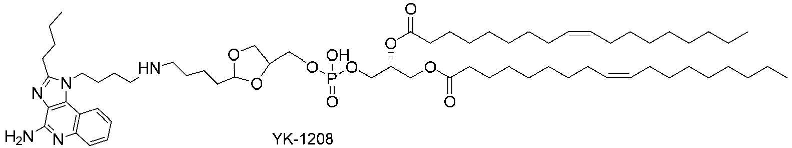 |
| Compound 23 | 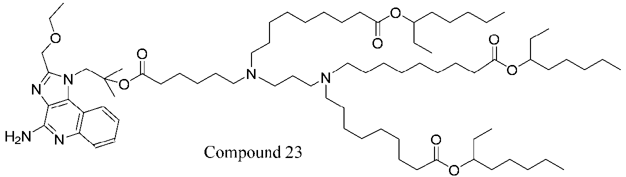 |
| 63-15 | 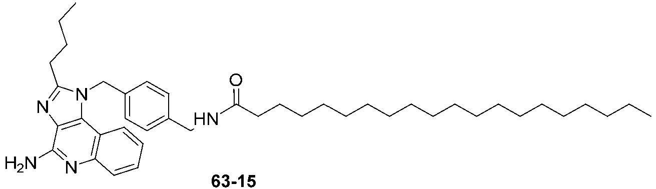 |
| TMX-201 | 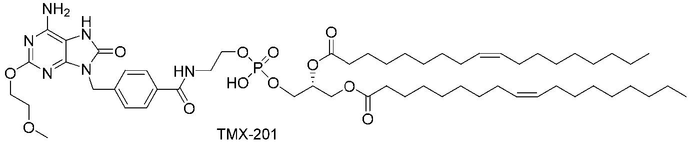 |

According to the preparation method in Example 3, the adjuvant lipid compound in Table 4 was used to partially replace DOTMA in the TLP composition at a ratio of 10 mol%, that is, the permanent anionic lipid ADOPE, the permanent cationic lipid DOTMA, the neutral lipid DOPE, and the adjuvant lipid were mixed at a molar ratio of 5:8:5:2 (*i.e.,* in the lipid composition, the molar percentages of the permanent anionic lipid, the permanent cationic lipid, the neutral lipid, and the adjuvant lipid were 25%, 40%, 25%, and 10%, respectively) to prepare an mRNA-TLP pharmaceutical composition. The results are shown in Table 5.

**Table 5: Particle size and PDI results of mRNA-TLP compositions containing different adjuvant lipids**

| No. | Name of adjuvant lipid | Particle size/nm | PDI |
|---|---|---|---|
| 1 | YK-1201 | 406.0 | 0.1749 |
| 2 | YK-1202 | 410.2 | 0.2359 |
| 3 | YK-1203 | 483.5 | 0.2606 |
| 4 | YK-1204 | 288.4 | 0.1878 |
| 5 | YK-1205 | 281.9 | 0.1433 |
| 6 | YK-1206 | 391.1 | 0.2101 |
| 7 | YK-1207 | 352.1 | 0.2231 |
| 8 | YK-1208 | 318.2 | 0.1586 |
| 9 | TLP (without adjuvant lipid) | 432.0 | 0.3000 |
| 10 | Compound 23 | 407.0 | 0.2854 |
| 11 | 63-15 | 292.6 | 0.2665 |
| 12 | TMX-201 | 320.0 | 0.2312 |

The results demonstrated that the adjuvant lipids YK-1201 to YK-1208 in Table 4 as well as Compound 23, 63-15, and TMX-201 were all able to prepare mRNA compositions with qualified particle size and PDI (a particle size controlled within 220 to 500 nm and a PDI of less than 0.5) according to the preparation method in Example 3.

### Example 5: Experiment on protein expression of mRNA-TLP composition prepared with adjuvant lipid in mice

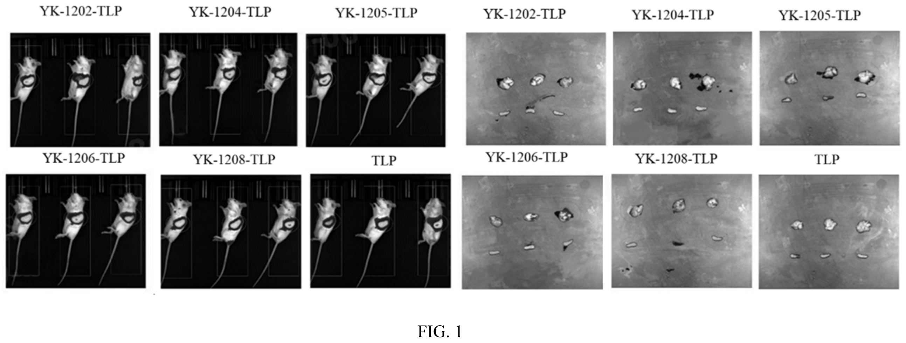

The Fluc-mRNA-TLP composition with or without adjuvant lipid prepared in Example 4 was injected into female BALB/c albino mice aged 4 to 6 weeks and weighing 17 to 19 g via the tail vein (at a dose of about 20 µg Fluc-mRNA/mouse). The mice were intraperitoneally injected with fluorescence imaging substrate 6 hours after administration. The mice were then allowed to move freely for 5 minutes, after which the total radiation intensity of the protein expressed by the mRNA contained in the mRNA composition in the mice (corresponding to the fluorescence intensity, *i.e.,* the protein expression level) was measured using an IVIS Spectrum small-animal *in vivo* imaging system. After sampling, the mice were euthanized by cervical dislocation and dissected to precisely isolate their internal organs (liver and spleen). The total radiation intensity of the protein expressed by Fluc-mRNA in various organs of the mice (corresponding to the fluorescence intensity, *i.e.,* the protein expression level) was measured using an IVIS Spectrum small-animal *in vivo* imaging system. The results of *in vivo* imaging and protein expression in various internal organs of the mice are shown in Table 6 and FIG. 1.

**Table 6: Experimental data of in vivo and organ imaging in mice**

| **No.** | **Adjuvant lipid in the composition** | **Particle size/nm** | **Total radiation intensity (× 10⁷ p/s)** | | | | |
|---|---|---|---|---|---|---|---|
| | | | ***In vivo* mice** | **Fold change of total radiation intensity *in vivo* relative to TLP** | **Liver** | **Spleen** | **Fold change of total radiation intensity in spleen relative to TLP** |
| 1 | YK-1201 | 406.0 | 0.98 | 0.9 | 0.11 | 1.41 | 1.0 |
| 2 | YK-1202 | 410.2 | 2.24 | 2.1 | 0.17 | 2.87 | 2.0 |
| 3 | YK-1203 | 483.5 | 1.07 | 1.0 | 0.13 | 1.58 | 1.1 |
| 4 | YK-1204 | 288.4 | 3.00 | 2.9 | 0.26 | 5.14 | 3.6 |
| 5 | YK-1205 | 281.9 | 2.30 | 2.2 | 0.08 | 3.18 | 2.2 |
| 6 | YK-1206 | 391.1 | 3.20 | 3.0 | 0.30 | 3.83 | 2.7 |
| 7 | YK-1207 | 352.1 | 0.81 | 0.8 | 0.22 | 1.53 | 1.1 |
| 8 | YK-1208 | 318.2 | 2.32 | 2.2 | 0.11 | 2.61 | 1.8 |
| 9 | TLP (without adjuvant lipid) | 432.0 | 1.05 | 1.0 | 0.11 | 1.42 | 1.0 |
| 10 | Compound 23 | 407.0 | 0.91 | 0.9 | 0.15 | 1.18 | 0.8 |
| 11 | 63-15 | 292.6 | 0.86 | 0.8 | 0.14 | 1.10 | 0.8 |
| 12 | TMX-201 | 320.0 | 1.02 | 1.0 | 0.12 | 1.20 | 0.8 |

It can be seen that by adding the adjuvant lipid of the present disclosure to the mRNA-TLP composition, mRNA can be efficiently delivered to the spleen of animals, and the delivery effect is significantly enhanced compared to the TLP composition without adjuvant lipid.

Compared with the addition of adjuvant lipids (Compound 23, 63-15, and TMX-201) in the prior art, the compositions with the addition of YK-1202, YK-1204, YK-1205, YK-1206, and YK-1208 of the present disclosure exhibited significantly enhanced total radiation intensity in spleen and total radiation intensity *in vivo.* For example, the composition with YK-1204 exhibited 4.4-fold, 4.7-fold, and 4.3-fold higher total radiation intensity in spleen compared to the compositions with Compound 23, 63-15, and TMX-201, respectively, and 3.3-fold, 3.5-fold, and 2.9-fold higher total radiation intensity *in vivo* compared to the compositions with Compound 23, 63-15, and TMX-201, respectively.

The compositions with structurally similar but different adjuvant lipids of the present disclosure showed significant differences in total radiation intensity in spleen and total radiation intensity *in vivo.* For example, the composition with YK-1206 exhibited 2.7-fold higher total radiation intensity in spleen and 3.3-fold higher total radiation intensity *in vivo,* respectively, compared to the composition with YK-1201.

### Example 6: Study on targeting of mRNA-TLP composition with adjuvant lipid to mouse spleen cells

1. The eGFP-mRNA-TLP composition with or without adjuvant lipid prepared in Example 3 was injected into female C57BL/6 mice aged 4 to 6 weeks and weighing 17 to 19 g via the tail vein (at a dose such that each mouse received about 80 µg of eGFP-mRNA). The mice were euthanized by cervical dislocation 24 hours after administration and dissected to precisely isolate the spleen.
2. Preparation of single cells
1) The isolated spleen tissue was ground and filtered through a cell strainer to obtain a single-cell suspension.
2) A 10-fold volume (about 4 mL) of red blood cell lysis buffer was added to lyse and remove red blood cells in the tissue.
3) Cell counting was performed, and 5 × 10⁶ cells were transferred to a flow cytometry tube (ensuring consistent cell numbers across samples).
3. Detection of immune cells in spleen tissue
1) 100 µL of surface antibody MIX was added to each single-cell suspension (see Table 7 for the components of surface antibody MIX), followed by incubation at room temperature in the dark for 15 minutes (one negative control).

**Table 7: Reagents and sources for flow cytometry of mouse spleen cells**

| **Reagent** | **Manufacturer** | **Volume (µL)** |
|---|---|---|
| PBS buffer | - | 100 |
| Zombie NIR^{™} Fixable Viability Kit (APC-A750) | Biolegend | 1 |
| PerCP anti-mouse CD45 | Biolegend | 1 |
| PE/Dazzle^{™} 594 anti-mouse CD3ε | Biolegend | 1 |
| PE/Cyanine7 anti-mouse NK-1.1 | BD | 1 |
| Brilliant Violet 605^{™} anti-mouse CD19 | Biolegend | 1 |
| Alexa Fluor^{®} 700 anti-mouse F4/80 | Biolegend | 1 |
| Pacific Blue^{™} anti-mouse/human CD11b | Biolegend | 1 |
| PE anti-mouse CD317 (BST2, PDCA-1) | Biolegend | 1 |

2) 2 mL of PBS was added, followed by centrifugation at 500 g for 5 minutes, and the supernatant was discarded.
3) The cells were resuspended in 200 µL of PBS, filtered through a 200-mesh nylon mesh filter, and analyzed using a Cytoflex S flow cytometer. The percentage of GFP-positive cells in each cell line was analyzed. The detection sequence for each cell line was as follows:
GFP proportion in T cells: CD45⁺→CD3⁺→GFP⁺
GFP proportion in B cells: CD45⁺→CD3⁻CD19⁺→GFP⁺
GFP proportion in NK cells: CD45⁺→NK1.1⁺→GFP⁺
GFP proportion in cDCs: CD45⁺→F4/80⁻CD11c⁺→GFP⁺
GFP proportion in pDCs: CD45⁺→F4/80⁻CD11c^{int}CD317⁺→GFP⁺
GFP proportion in macrophages: CD45⁺→F4/80⁺→GFP⁺

### Experimental results:

The percentages of eGFP-positive cells in mouse spleen cells are shown in Table 8.

**Table 8: Percentage of eGFP-positive cells in mouse spleen cells**

| **No.** | **Name of adjuvant lipid** | **Particle size/nm** | **Percentage of eGFP-positive cells/%** | | | | | | | | | | | |
|---|---|---|---|---|---|---|---|---|---|---|---|---|---|---|
| | | | **T cells** | **FC vs. TLP** | **B cells** | **FC vs. TLP** | **NK cells** | **FC vs. TLP** | **cDCs** | **FC vs. TLP** | **pDCs** | **FC vs. TLP** | **Macrophages** | **FC vs. TLP** |
| 1 | Blank control | 258.4 | 0.11 | - | 0.02 | - | 0.34 | - | 0.46 | - | 0.74 | - | 0.07 | - |
| 2 | YK-1201 | 406.0 | 0.76 | 1.0 | 0.19 | 0.9 | 2.61 | 0.9 | 5.51 | 1.1 | 6.39 | 1.0 | 6.60 | 1.0 |
| 3 | YK-1202 | 410.2 | 0.79 | 1.1 | 0.26 | 1.2 | 3.98 | 1.4 | 7.04 | 1.4 | 8.52 | 1.4 | 7.29 | 1.1 |
| 4 | YK-1203 | 483.5 | 0.55 | 0.7 | 0.20 | 1.0 | 2.47 | 0.8 | 5.26 | 1.0 | 5.88 | 1.0 | 5.52 | 0.9 |
| 5 | YK-1204 | 288.4 | 0.98 | 1.3 | 0.47 | 2.1 | 4.89 | 1.7 | 8.54 | 1.7 | 10.46 | 1.7 | 11.04 | 1.7 |
| 6 | YK-1205 | 281.9 | 1.37 | 1.9 | 0.34 | 1.5 | 3.97 | 1.4 | 8.84 | 1.7 | 9.52 | 1.5 | 9.35 | 1.5 |
| 7 | YK-1206 | 391.1 | 1.28 | 1.7 | 0.43 | 2.0 | 4.81 | 1.7 | 9.54 | 1.9 | 10.12 | 1.6 | 10.59 | 1.7 |
| 8 | YK-1207 | 352.1 | 0.65 | 0.9 | 0.21 | 1.0 | 2.62 | 0.9 | 5.64 | 1.1 | 6.22 | 1.0 | 6.22 | 1.0 |
| 9 | YK-1208 | 318.2 | 0.87 | 1.2 | 0.39 | 1.8 | 4.20 | 1.4 | 8.12 | 1.6 | 7.95 | 1.3 | 8.43 | 1.3 |
| 10 | TLP (without adjuvant lipid) | 432.0 | 0.74 | 1.0 | 0.22 | 1.0 | 2.91 | 1.0 | 5.06 | 1.0 | 6.22 | 1.0 | 6.40 | 1.0 |
| 11 | Compound 23 | 407.0 | 0.55 | 0.7 | 0.23 | 1.0 | 2.51 | 0.9 | 4.93 | 1.0 | 5.57 | 0.9 | 4.96 | 0.8 |
| 12 | 63-15 | 292.6 | 0.73 | 1.0 | 0.16 | 0.7 | 1.73 | 0.6 | 5.14 | 1.0 | 5.82 | 0.9 | 6.13 | 1.0 |
| 13 | TMX-201 | 320.0 | 0.81 | 1.1 | 0.25 | 1.1 | 3.12 | 1.1 | 5.65 | 1.1 | 6.82 | 1.1 | 6.51 | 1.0 |

Antigen-presenting cells (also referred to as "APCs") are a class of immune cells capable of capturing and processing antigens, and presenting the processed antigens to lymphocytes, including dendritic cells (DCs), B lymphocytes, and macrophages, which play a crucial role in the human body, primarily involving immune recognition, immune response, and immune regulation.

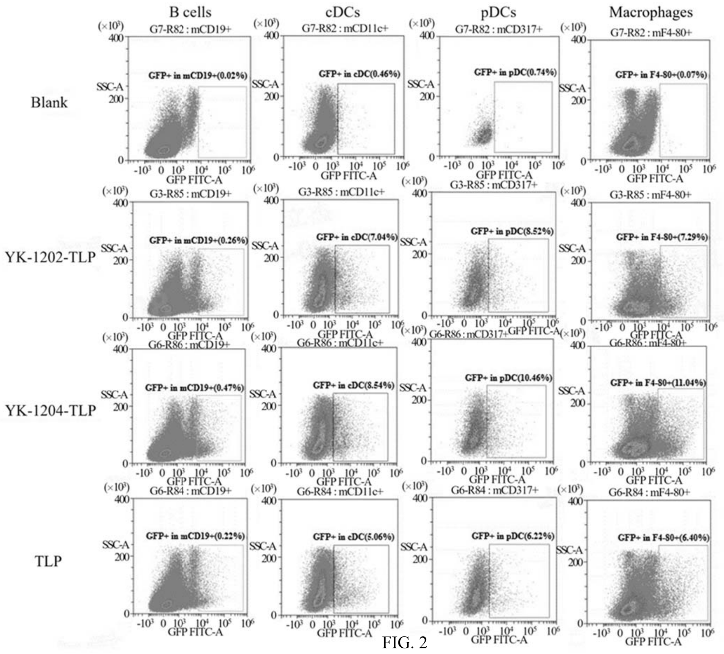

As can be seen from the data in Table 8 and FIG. 2, the mRNA-TLP compositions prepared using the adjuvant lipids of the present disclosure significantly increased the percentage of antigen-presenting cells expressing antigens in the spleen compared to the TLP-mRNA composition without adjuvant. For example, the mRNA-TLP composition prepared with YK-1204 increased the percentage of eGFP-positive cells in B cells, cDCs, pDCs, and macrophages in the spleen by 2.1-fold, 1.7-fold, 1.7-fold, and 1.7-fold, respectively.

Compared with the adjuvants in the prior art, the mRNA-TLP compositions prepared using the adjuvant lipids of the present disclosure exhibited significantly enhanced targeting to antigen-presenting cells in mouse spleen. For example, the percentages of eGFP-positive cells in B cells, cDCs, pDCs, and macrophages in mouse spleen for YK-1204 were 1.9-fold, 1.5-fold, 1.5-fold, and 1.7-fold higher than those for TMX-201, respectively.

The mRNA-TLP compositions prepared with different adjuvant lipids of the present disclosure showed significant differences in targeting to antigen-presenting cells in mouse spleen, with the data for YK-1202, YK-1204, YK-1205, YK-1206, and YK-1208 being significantly higher than those for other compounds. For example, the mRNA-TLP composition prepared with YK-1206 exhibited 2.2-fold, 1.8-fold, 1.7-fold, and 1.9-fold higher percentages of eGFP-positive cells in B cells, cDCs, pDCs, and macrophages, respectively, compared to the mRNA-TLP composition prepared with YK-1203.

### Example 7: Study on maturation status of antigen-presenting cells in mouse spleen using mRNA-TLP composition prepared with adjuvant lipid

CD86 (cluster of differentiation 86) is a molecule expressed on antigen-presenting cells, which provides co-stimulatory signals required for T cell activation and survival. The maturation status of antigen-presenting cells in the spleen can be indicated by the upregulation of CD86. In this example, based on the flow cytometry experiment in Example 5, an additional experiment was conducted to detect the upregulation of CD86. The surface antibody used was Brilliant Violet 510^{™} anti-mouse CD86. The detection sequence for each cell line was as follows:
CD86 proportion in B cells: CD45⁺→CD3⁻CD19⁺→CD86⁺
CD86 proportion in NK cells: CD45⁺→NK1.1⁺→CD86⁺
CD86 proportion in cDCs: CD45⁺→F4/80⁻CD11c⁺→CD86⁺
CD86 proportion in pDCs: CD45⁺→F4/80⁻CD11c^{int}CD317⁺→CD86⁺
CD86 proportion in macrophages: CD45⁺→F4/80⁺→CD86⁺

### Experimental results:

The percentages of CD86-positive cells in mouse spleen cells are shown in Table 9.

**Table 9: Percentage of CD86-positive cells in mouse spleen cells**

| **No.** | **Name of adjuvant lipid** | **Particle size/nm** | **Percentage of CD86-positive cells/%** | | | | | | | | | |
|---|---|---|---|---|---|---|---|---|---|---|---|---|
| | | | **B cells** | **FC vs. TLP** | **NK cells** | **FC vs. TLP** | **cDCs** | **FC vs. TLP** | **pDCs** | **FC vs. TLP** | **Macrophages** | **FC vs. TLP** |
| 1 | Blank control | 258.4 | 0.14 | - | 5.10 | - | 8.88 | - | 20.90 | - | 2.87 | - |
| 2 | YK-1201 | 406.0 | 0.82 | 1.3 | 14.95 | 1.7 | 45.77 | 1.2 | 73.44 | 1.4 | 21.05 | 1.4 |
| 3 | YK-1202 | 410.2 | 1.01 | 1.6 | 21.23 | 2.4 | 55.66 | 1.4 | 80.11 | 1.6 | 22.58 | 1.5 |
| 4 | YK-1203 | 483.5 | 0.74 | 1.2 | 11.50 | 1.3 | 48.35 | 1.3 | 77.93 | 1.5 | 20.99 | 1.4 |
| 5 | YK-1204 | 288.4 | 0.97 | 1.5 | 22.10 | 2.5 | 65.77 | 1.7 | 81.00 | 1.6 | 24.28 | 1.6 |
| 6 | YK-1205 | 281.9 | 0.73 | 1.1 | 22.56 | 2.5 | 57.82 | 1.5 | 83.68 | 1.6 | 25.68 | 1.7 |
| 7 | YK-1206 | 391.1 | 1.19 | 1.9 | 23.51 | 2.6 | 64.43 | 1.7 | 83.18 | 1.6 | 22.76 | 1.5 |
| 8 | YK-1207 | 352.1 | 0.66 | 1.0 | 14.49 | 1.6 | 49.76 | 1.3 | 68.95 | 1.4 | 19.76 | 1.3 |
| 9 | YK-1208 | 318.2 | 0.96 | 1.5 | 21.47 | 2.4 | 56.65 | 1.5 | 79.42 | 1.6 | 23.83 | 1.6 |
| 10 | TLP (without adjuvant lipid) | 432.0 | 0.64 | 1.0 | 8.93 | 1.0 | 38.40 | 1.0 | 50.96 | 1.0 | 15.33 | 1.0 |
| 11 | Compound 23 | 407.0 | 0.53 | 0.8 | 9.52 | 1.1 | 30.52 | 0.8 | 55.12 | 1.1 | 18.21 | 1.2 |
| 12 | 63-15 | 292.6 | 0.65 | 1.0 | 8.45 | 0.9 | 29.18 | 0.8 | 58.63 | 1.2 | 16.58 | 1.1 |
| 13 | TMX-201 | 320.0 | 0.56 | 0.9 | 8.62 | 1.0 | 30.95 | 0.8 | 50.69 | 1.0 | 18.19 | 1.2 |

It can be seen that the mRNA-TLP compositions prepared using the adjuvant lipids of the present disclosure significantly increased the percentage of CD86-positive antigen-presenting cells in the spleen. Compared with the mRNA-TLP composition without adjuvant, the mRNA-TLP compositions prepared using the adjuvant lipids YK-1202, YK-1204 to YK-1206, and YK-1208 of the present disclosure significantly enhanced the maturation status of antigen-presenting cells in mouse spleen. For example, the mRNA-TLP composition with YK-1205 exhibited 1.1-fold, 1.5-fold, 1.6-fold, and 1.7-fold higher percentages of CD86-positive cells in B cells, cDCs, pDCs, and macrophages in mouse spleen, respectively, compared to the TLP composition without adjuvant lipid.

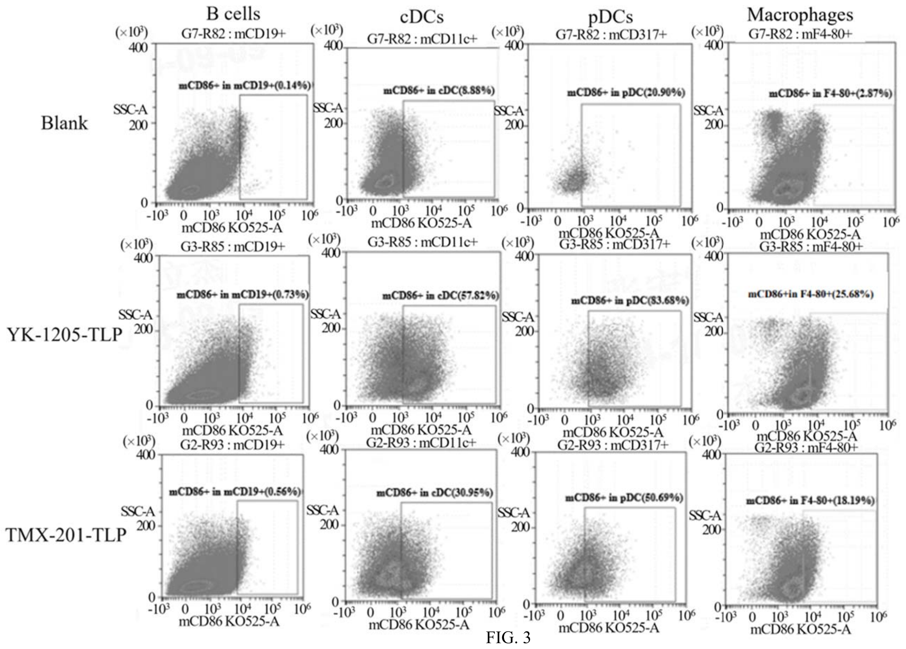

Compared with the adjuvants in the prior art, the mRNA-TLP compositions prepared using the adjuvant lipids YK-1202, YK-1204, YK-1205, YK-1206, and YK-1208 of the present disclosure significantly enhanced the maturation status of antigen-presenting cells in mouse spleen. For example, the mRNA-TLP composition prepared with YK-1205 exhibited 1.3-fold, 1.9-fold, 1.7-fold, and 1.4-fold higher percentages of CD86-positive cells in B cells, cDCs, pDCs, and macrophages in mouse spleen, respectively, compared to that prepared with TMX-201. (FIG. 3)

The mRNA-TLP compositions prepared with different adjuvant lipids provided by the present disclosure showed significant differences in the percentage of CD86-positive antigen-presenting cells in the spleen. For example, the mRNA-TLP composition prepared with YK-1206 exhibited a 1.8-fold higher percentage of CD86-positive B cells in mouse spleen compared to that prepared with YK-1207.

### Example 8: Study on T cell activation in mouse spleen using mRNA-TLP composition prepared with adjuvant lipid

CD69 is a marker of T cell activation and one of the earliest markers to be upregulated after T cell activation. T cell activation in the spleen can be indicated by the upregulation of CD69. In this example, based on the flow cytometry experiment in Example 5, an additional experiment was conducted to detect the upregulation of CD69. The surface antibody used was APC anti-mouse CD69. The detection sequence for the cell line was as follows:
CD69 proportion in T cells: CD45⁺→CD3⁺→CD69⁺

### Experimental results:

The percentages of CD69-positive T cells in mouse spleen are shown in Table 10.

**Table 10: Percentage of CD69-positive T cells in mouse spleen**

| **No.** | **Name** | **Particle size/nm** | **Percentage of CD69-positive cells/%** | **FC vs. TLP** |
|---|---|---|---|---|
| 1 | Blank control | 258.4 | 5.59 | - |
| 2 | YK-1201 | 406.0 | 59.08 | 1.3 |
| 3 | YK-1202 | 410.2 | 68.77 | 1.5 |
| 4 | YK-1203 | 483.5 | 57.95 | 1.3 |
| 5 | YK-1204 | 288.4 | 75.86 | 1.7 |
| 6 | YK-1205 | 281.9 | 75.90 | 1.7 |
| 7 | YK-1206 | 391.1 | 73.21 | 1.6 |
| 8 | YK-1207 | 352.1 | 55.44 | 1.2 |
| 9 | YK-1208 | 318.2 | 69.05 | 1.6 |
| 10 | TLP (without adjuvant lipid) | 432.0 | 44.39 | 1.0 |
| 11 | Compound 23 | 407.0 | 48.13 | 1.1 |
| 12 | 63-15 | 292.6 | 49.54 | 1.1 |
| 13 | TMX-201 | 320.0 | 30.46 | 0.7 |

The mRNA-TLP compositions prepared using the adjuvant lipids of the present disclosure significantly increased the percentage of CD69-positive T cells in the spleen compared to the mRNA-TLP composition without adjuvant lipid. For example, the mRNA-TLP compositions prepared using the adjuvant lipids YK-1202, YK-1204, YK-1205, YK-1206, and YK-1208 of the present disclosure exhibited 1.5-fold, 1.7-fold, 1.7-fold, 1.6-fold, and 1.6-fold higher percentages of CD69-positive T cells in the spleen, respectively, compared to the TLP composition without adjuvant, showing a significant improvement.

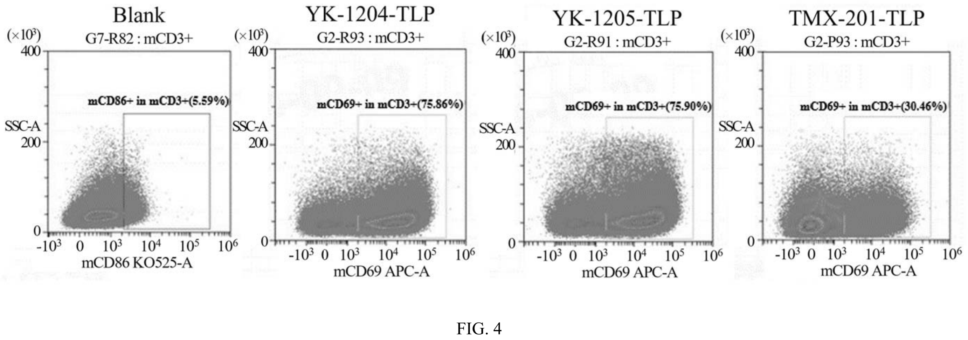

Compared with the adjuvant lipids in the prior art, the mRNA-TLP compositions prepared using the adjuvant lipids of the present disclosure significantly enhanced the activation status of T cells in mouse spleen. For example, the mRNA-TLP composition prepared with YK-1205 exhibited a 2.5-fold higher percentage of CD69-positive T cells in mouse spleen compared to the mRNA-TLP composition prepared with TMX-201. (FIG. 4)

The mRNA-TLP compositions prepared with different adjuvant lipids of the present disclosure showed significant differences in the percentage of CD69-positive T cells in mouse spleen. For example, the mRNA-TLP compositions prepared with YK-1202, YK-1204, YK-1205, YK-1206, and YK-1208 exhibited significantly higher percentages of CD69-positive T cells in mouse spleen compared to other compositions. Specifically, the mRNA-TLP composition prepared with YK-1205 exhibited the highest percentage of CD69-positive T cells, which was 1.4-fold higher than that prepared with YK-1207 (the lowest).

### Example 9: Induction of cytokines IFN-γ and IL-12 by mRNA-TLP compositions containing adjuvant lipids

Interferon-γ (IFN-γ) is an important cytokine primarily produced by activated T cells and natural killer (NK) cells. It plays a critical role in immune responses, exhibiting antiviral, antitumor, immunomodulatory, and pro-inflammatory functions, and can be used to treat a variety of diseases. Interleukin-12 (IL-12) is a multifunctional cytokine produced by dendritic cells, macrophages, B lymphocytes, and other antigen-presenting cells. It plays a significant role in regulating immune responses, promoting Th1 immunity, enhancing cytotoxicity, and contributing to tumor immunity. Assessing the induction of cytokines IFN-γ and IL-12 by mRNA-TLP compositions containing adjuvant lipids can reflect the improvement of the adjuvant lipids on the innate immunity of the mRNA-TLP composition.

### Experimental procedure:

8-week-old female C57BL/6J mice were injected with the OVA-mRNA-TLP (40 µg) composition prepared according to Example 4 via the tail vein. After 6 hours, the mice were euthanized by enucleation, and blood was collected to obtain as much serum as possible. The levels of IFN-γ and IL-12 in the serum were measured by ELISA. Mice injected with an equal volume of blank lipid solution served as the blank control group.

ELISA assay: The levels of IFN-γ and IL-12 in mouse serum were measured by standard ELISA according to the manufacturer's instructions.

**Table 11: Comparison of induction of cytokines IFN-γ and IL-12 by mRNA-TLP compositions containing adjuvant lipids**

| **Group** | **Cytokine/pg/mL** | |
|---|---|---|
| | **IFN-γ** | **IL-12** |
| | Mean | Mean |
| TLP (without adjuvant lipid) | 136.69 | 377.25 |
| YK-1202-TLP | 245.23 | 575.22 |
| YK-1204-TLP | 363.45 | 1164.71 |
| YK-1205-TLP | 278.24 | 683.83 |
| YK-1208-TLP | 293.86 | 707.74 |

### Experimental results:

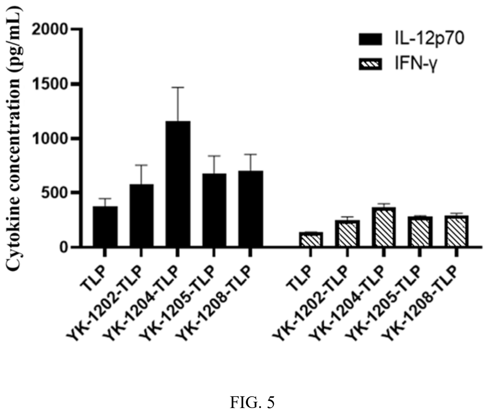

As shown in Table 11 and FIG. 5, the mRNA-TLP compositions containing adjuvant lipids exhibited a significant increase in cytokines IFN-γ and IL-12 in serum 6 hours after injection compared to the mRNA-TLP composition without adjuvant lipid. Specifically, the levels of IFN-γ and IL-12 induced by YK-1202-TLP were 1.8-fold and 1.5-fold higher, respectively, than those induced by the mRNA-TLP composition without adjuvant lipid; the levels of IFN-γ and IL-12 induced by YK-1204-TLP were 2.7-fold and 3.1-fold higher, respectively, than those induced by the mRNA-TLP composition without adjuvant lipid; the levels of IFN-γ and IL-12 induced by YK-1205-TLP were 2.0-fold and 1.8-fold higher, respectively, than those induced by the mRNA-TLP composition without adjuvant lipid; the levels of IFN-γ and IL-12 induced by YK-1208-TLP were 2.1-fold and 1.9-fold higher, respectively, than those induced by the mRNA-TLP composition without adjuvant lipid.

The experimental results demonstrated that by measuring the levels of cytokines IFN-γ and IL-12 induced by the mRNA-TLP compositions containing adjuvant lipids, it can be proven that the adjuvant lipids of the present disclosure can initiate an immunostimulatory program and significantly enhance the innate immunity of the mRNA-TLP composition.

### Example 10: Therapeutic effect of mRNA-TLP compositions containing adjuvant lipids on tumor-bearing mouse model

### Experimental procedure:

1) Establishment of B16F10-OVA mouse model: Female C57BL/6J mice aged 6 to 8 weeks were acclimated for at least three days prior to the study. The mice had free access to food and sterile water and were housed under a 12-hour light/dark cycle at 22°C ± 2°C and 55% ± 15% relative humidity. B16F10-OVA cells (Zhejiang Meisen Cell Technology Co., Ltd.) were cultured in complete medium as described in the instructions for B16F10-OVA (CTCC-001-0727) at 37°C with 5% CO₂. The cells were harvested using 0.25% trypsin-EDTA, then resuspended in Dulbecco's phosphate-buffered saline (DPBS), and transplanted into female C57BL/6J mice at 2 × 10⁵ cells/100 µL/mouse (B16F10-OVA) by subcutaneous (SC) injection to establish a subcutaneous B16F10-OVA tumor model. Vaccination was initiated when the tumor volume reached approximately 100 mm³.
2) Vaccination: For the B16F10-OVA mouse model, the enrolled C57BL/6J mice were vaccinated with OVA mRNA YK-1202-TLP, YK-1204-TLP, YK-1205-TLP, and YK-1208-TLP compositions (40 µg) containing TLR adjuvant lipids prepared according to Example 3 via tail vein injection on days 3, 7, 10, 12, 14, and 17 after cell injection (day 0) (each mouse received a vaccine containing 40 µg of therapeutic mRNA-OVA per injection). Mice vaccinated with an equal volume of OVA mRNA-TLP without adjuvant served as the control group, with 8 mice per group in parallel.
3) Tumor size and survival rate: Starting from day 7 after tumor inoculation, tumor diameters were measured three times per week. The tumor volume of C57BL/6J mice was calculated using the following formula: V (mm³) = x × y²/2, where V represents the tumor volume, x represents the long diameter of the tumor, and y represents the short diameter of the tumor, with units in mm. Changes in body weight of C57BL/6J mice were recorded three times per week using an electronic balance, and survival rates were statistically analyzed.

### Experimental results:

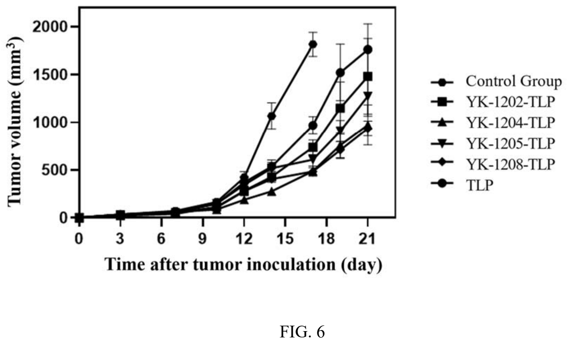

As shown in FIG. 6 and Table 12, on day 10 after tumor inoculation, mice in the blank lipid control group (Control Group) and the non-adjuvant OVA mRNA-TLP composition group entered a rapid tumor growth phase, but the growth rate in the TLP group was significantly lower than that in the blank control group. Correspondingly, the OVA mRNA pharmaceutical compositions with adjuvant lipids all showed significant tumor growth delay. Starting from day 14, compared to the TLP group, the tumor volumes in the mRNA-TLP pharmaceutical compositions with adjuvant lipids also exhibited stronger tumor growth inhibition, with the mRNA-TLP compositions prepared with YK-1204 and YK-1208 showing relatively better tumor suppression effects.

**Table 12: Tumor growth in mice during treatment with different compositions**

| Day n | Tumor volume (mean)/mm³ | | | | | |
|---|---|---|---|---|---|---|
| | Blank lipid | TLP | YK-1202-TLP | YK-1204-TLP | YK-1205-TLP | YK-1208-TLP |
| 3 | 33.45 | 33.45 | 20.2 | 33.2 | 30.21 | 30.1 |
| 7 | 69.79 | 61.79 | 39.96 | 60.49 | 52.86 | 54.55 |
| 10 | 163.45 | 143.45 | 111.96 | 85.68 | 148.43 | 107.3 |
| 12 | 419.86 | 369.86 | 281.9 | 188.4 | 344.05 | 278.64 |
| 14 | 1068.38 | 534.19 | 428.14 | 276.2 | 517.14 | 406.98 |
| 17 | 1815.82 | 968.33 | 738.68 | 486.84 | 608.3 | 482.53 |
| 19 | / | 1522.21 | 1150.07 | 759.42 | 908.79 | 708.71 |
| 21 | / | 1762.42 | 1481.72 | 972.66 | 1275.11 | 936.83 |

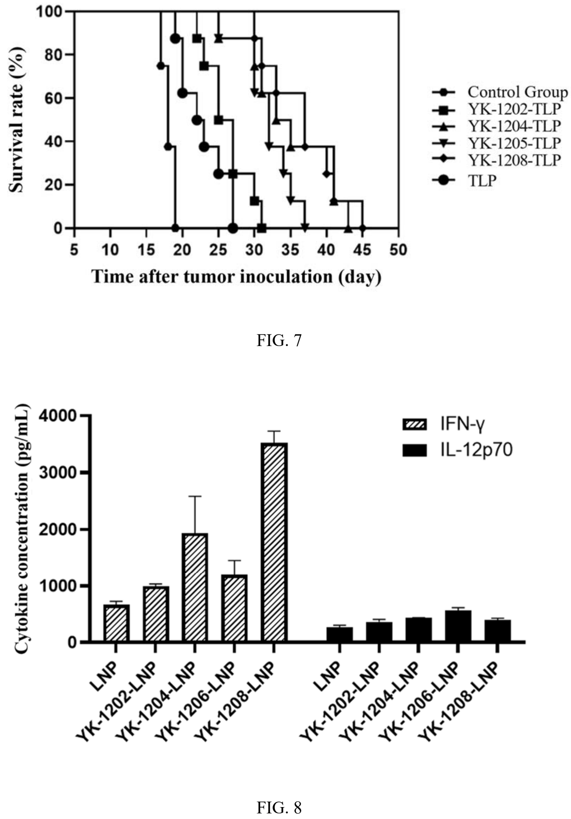

In terms of survival rate, as shown in FIG. 7, the mice in the blank lipid control group started to die on day 17 after tumor inoculation, with all mice dead on day 19; the mice in the non-adjuvant TLP group started to die on day 19, with all mice dead on day 27; the mice injected with the mRNA-TLP compositions containing adjuvant lipids YK-1202, YK-1204, YK-1205, and YK-1208 started to die on days 23, 25, 25, and 30, respectively, with all mice dead on days 31, 43, 37, and 45, respectively. It can be seen that the addition of adjuvant lipids significantly prolonged the survival time of mice.

### Example 11: Effect of different amounts of adjuvant lipids on LNP-mRNA composition

The effect of adjuvant lipids on LNP compositions was investigated in this example.

### Experimental procedure:

YK-009, YK-1202, DSPC, cholesterol, and DMG-PEG2000 were weighed according to the molar ratios in Table 12 and dissolved in ethanol to prepare an ethanol lipid solution. The eGFP-mRNA was diluted in citrate buffer (pH = 4 to 5) to obtain an mRNA aqueous solution. The ethanol lipid solution was mixed with the Fluc-mRNA aqueous solution prepared from different cap structures at a volume ratio of 1:3 using a microfluidic device at a flow rate of 10 mL/min to prepare LNPs at a weight ratio of total lipid to mRNA of approximately 15:1. The resulting liposomes were diluted 10-fold with PBS, and then ultrafiltered with a 300 KDa ultrafiltration tube to remove ethanol. The liposome solution was then adjusted to a certain volume with PBS. Finally, the LNPs were filtered through a 0.2 µm sterile filter to obtain an LNP pharmaceutical composition with or without adjuvant lipid. The particle size and polydispersity index (PDI) were determined by dynamic light scattering using a Malvern laser particle size analyzer. 10 µL of the liposome solution was taken, diluted to 1 mL with RNase-free deionized water, and added to a sample pool. Each sample was measured in triplicate. The measurement conditions were: a scattering angle of 90° and a temperature of 25°C. The encapsulation efficiency of LNPs was determined using the Quant-iT RiboGreen RNA Quantification Kit (Thermo Fisher Scientific, UK) according to the manufacturer's instructions.

**Table 13: Particle size, PDI, and encapsulation efficiency of compositions with different formulations**

| No. | YK-009 | DSPC | Cholesterol | DMG-PEG2000 | YK-1202 | Particle size/nm | PDI | Encapsulation efficiency/% |
|---|---|---|---|---|---|---|---|---|
| 1 | 47.5 mol% | 10 mol% | 38.5 mol% | 1.5 mol% | 2.5 mol% | 85.1 | 0.12 | 88.4% |
| 2 | 45 mol% | 10 mol% | 38.5 mol% | 1.5 mol% | 5 mol% | 87.4 | 0.14 | 90.2% |
| 3 | 40 mol% | 10 mol% | 38.5 mol% | 1.5 mol% | 10 mol% | 76.5 | 0.05 | 95.1% |
| 4 | 35 mol% | 10 mol% | 38.5 mol% | 1.5 mol% | 15 mol% | 92.1 | 0.16 | 89.6% |
| 5 | 30 mol% | 10 mol% | 38.5 mol% | 1.5 mol% | 20 mol% | 95.2 | 0.11 | 85.4% |

The results demonstrated that when YK-1202 was used to partially replace the cationic lipid YK-009 in the LNP composition at 2.5%, 5.0%, 10.0%, 15.0%, and 20.0%, the particle size, PDI, and encapsulation efficiency of the resulting LNP pharmaceutical composition were within the qualified range (a particle size of less than 200 nm, a PDI of less than 0.3, and an encapsulation efficiency of more than 85%). The particle size, PDI, and encapsulation efficiency were optimal when the adjuvant was at 10 mol%. Subsequently, the adjuvant lipid was studied at a 10% adjuvant lipid ratio.

### Example 12: Effect of different adjuvant lipids on LNP-mRNA composition

### Experimental procedure:

According to the preparation method in Example 11, the adjuvant lipid compound in Table 4 was used to partially replace YK-009 in the LNP composition at a ratio of 10 mol%, that is, YK-009, DSPC, cholesterol, DMG-PEG2000, and the adjuvant lipid were mixed at a molar ratio of 40:10:38.5:1.5:10 to prepare a Fluc-mRNA-LNP pharmaceutical composition. Evaluation method: First, the particle size, polydispersity index (PDI), and encapsulation efficiency were determined according to the method in Example 11. Then, the pharmaceutical composition was added to the cell culture medium in a 96-well plate, and after further incubation for 24 hours, the corresponding reagents were added according to the instructions of the Gaussia Luciferase Assay Kit. The fluorescence intensity of each well was measured by the IVIS fluorescence imaging system. Finally, 10 µL of CCK-8 solution was added to each well of the aforementioned plate after 24 hours of incubation, and the plate was incubated in an incubator for 1 hour. The absorbance at 450 nm was measured using a microplate reader to determine the cell viability. The results are shown in Table 14.

**Table 14: Particle size and PDI results of LNP compositions containing different adjuvants**

| No. | Name of adjuvant lipid | Particle size/nm | PDI | Encapsulation efficiency/% | Relative fluorescence intensity/RLU | Cell viability/% |
|---|---|---|---|---|---|---|
| 1 | YK-1201 | 73.2 | 0.13 | 91.5 | 535355 | 53 |
| 2 | YK-1202 | 67.5 | 0.08 | 95.1 | 1736677 | 83 |
| 3 | YK-1203 | 72.3 | 0.11 | 91.2 | 437834 | 61 |
| 4 | YK-1204 | 78.6 | 0.11 | 94.3 | 2758655 | 88 |
| 5 | YK-1205 | 78.2 | 0.12 | 95.2 | 2167544 | 87 |
| 6 | YK-1206 | 87.2 | 0.08 | 95.2 | 2624445 | 91 |
| 7 | YK-1207 | 75.3 | 0.13 | 93.2 | 213475 | 65 |
| 8 | YK-1208 | 87.5 | 0.11 | 96.2 | 2263566 | 84 |
| 9 | LNP (without adjuvant lipid) | 77.5 | 0.09 | 95.1 | 1474665 | 86 |
| 10 | 63-15 | 76.8 | 0.10 | 94.7 | 1453837 | 84 |
| 11 | TMX-201 | 77.9 | 0.11 | 95.3 | 1574333 | 83 |

The results demonstrated that the adjuvant lipids YK-1201 to 1208 of the present disclosure and the adjuvant lipids 63-15 and TMX-201 disclosed in the prior art can be used to prepare favorable mRNA-LNP compositions by replacing YK-009 at a ratio of 10%. All LNPs had a particle size of 67 to 88 nm, a PDI of 0.08 to 0.13, and an encapsulation efficiency of 90% or more.

However, the LNP compositions prepared above showed significant differences in the relative fluorescence intensity (mRNA translation efficiency) and cell viability (cytotoxicity). The relative fluorescence intensity of the mRNA-LNP compositions prepared with YK-1202, YK-1204, YK-1205, YK-1206, and YK-1208 was significantly higher than that of the YK-1201, YK-1203, and YK-1207 groups, and was also significantly higher than that of the LNP composition without adjuvant lipid and the mRNA-LNP compositions with 63-15 and TMX-201. Moreover, the cytotoxicity of the mRNA-LNP compositions prepared with YK-1202, YK-1204, YK-1205, YK-1206, and YK-1208 was significantly lower than that of the YK-1201, YK-1203, and YK-1207 groups, and was comparable to that of the mRNA-LNP composition without adjuvant lipid and the mRNA-LNP compositions with 63-15 and TMX-201.

### Example 13: In vivo expression of LNP-mRNA compositions with different adjuvant lipids

### Experimental procedure:

The LNP preparation containing 10 µg of Fluc-mRNA with different adjuvant lipids prepared according to Example 11 was injected into female BALB/C mice aged 4 to 6 weeks and weighing 17 to 19 g via the tail muscle. The mice were intraperitoneally injected with fluorescence imaging substrate at specific time points (6 hours) after administration. The mice were then allowed to move freely for 5 minutes, after which the average radiation intensity of the protein expressed by the mRNA contained in the LNPs in the mice (corresponding to the fluorescence intensity) was measured using an IVIS Spectrum small-animal *in vivo* imaging system.

After sampling, the mice were euthanized with carbon dioxide and dissected to precisely isolate their internal organs (liver, spleen, and lungs). The total radiation intensity of the protein expressed by the mRNA contained in the LNPs in various organs of the mice (corresponding to the fluorescence intensity) was measured using an IVIS Spectrum small-animal *in vivo* imaging system. The results of *in vivo* imaging in mice are shown in Table 15.

**Table 15: Experimental data of in vivo imaging in mice at specific time points (6 hours) after administration**

| Adjuvant lipid | Total radiation intensity in mice (× 10⁸ p/s) | | | | |
|---|---|---|---|---|---|
| | Injection site | Abdominal cavity | Liver | Spleen | Lung |
| YK-1201 | 1.28 | 5.33 | 4.63 | 0.22 | 0.01 |
| YK-1202 | 2.45 | 8.34 | 8.24 | 0.63 | 0.01 |
| YK-1204 | 3.27 | 9.45 | 10.35 | 0.85 | 0.01 |
| YK-1205 | 3.33 | 10.63 | 10.83 | 0.96 | 0.01 |
| YK-1206 | 3.56 | 11.23 | 11.72 | 0.84 | 0.01 |
| YK-1208 | 2.55 | 8.51 | 7.66 | 0.68 | 0.01 |
| LNP (without adjuvant lipid) | 2.04 | 6.37 | 7.11 | 0.44 | 0.01 |
| TMX-201 | 2.12 | 7.30 | 7.04 | 0.46 | 0.01 |

### Experimental results:

It can be seen that the LNP preparations prepared with YK-1202, YK-1204, YK-1205, YK-1206, and YK-1208 significantly enhanced the expression of delivered mRNA in the injection site, abdominal cavity, liver, and spleen of mice compared to LNPs without adjuvant lipid or those containing the representative adjuvant lipid TMX-201 in the prior art. For example, the mRNA-LNP preparation with YK-1206 exhibited 1.7-fold higher expression in the injection site of mice compared to LNPs without adjuvant lipid, and 1.8-fold higher expression in the spleen of mice compared to LNPs with TMX-201.

There are abundant APC cells in the spleen and muscle (injection site). By increasing the expression of delivered mRNA in the spleen and muscle, the mRNA vaccine can rapidly induce immune responses and antibody production *in vivo.* The preventive and therapeutic efficacy can be significantly improved without altering vaccine components, holding substantial clinical significance.

Furthermore, the Fluc-mRNA-containing LNP preparations with adjuvant lipids showed significant differences in the expression across different organs of mice. LNPs prepared with YK-1202, YK-1204, YK-1205, YK-1206, YK-1208, TMX-201, or without adjuvant lipid all showed expression in the liver and spleen but none in the lungs.

Compared with the structurally similar adjuvant lipid YK-1201, the mRNA-LNP preparations prepared with YK-1202, YK-1204, YK-1205, YK-1206, and YK-1208 exhibited significantly enhanced mRNA expression in the liver and spleen of mice. For example, the LNP preparation with YK-1206 exhibited 2.8-fold higher mRNA expression in the injection site and 3.8-fold higher expression in the spleen compared to the LNP preparation with YK-1201.

### Example 14: Induction of cytokines IFN-γ and IL-12 by mRNA-LNP compositions containing adjuvant lipids

### Experimental procedure:

8-week-old female C57BL/6J mice were intramuscularly injected with the OVA-mRNA-LNP (5 µg) composition prepared according to Example 11. After 6 hours, the mice were euthanized by enucleation, and blood was collected to obtain as much serum as possible. The levels of IFN-γ and IL-12 in the serum were measured by ELISA. Mice injected with an equal volume of blank lipid solution served as the blank control group.

ELISA assay: The levels of IFN-γ and IL-12 in mouse serum were measured by standard ELISA according to the manufacturer's instructions.

**Table 16: Comparison of induction of cytokines IFN-γ and IL-12 by mRNA-LNP compositions containing adjuvant lipids**

| **Group** | **Cytokine/pg/mL** | |
|---|---|---|
| | **IL-12** | **IFN-γ** |
| | Mean | Mean |
| LNP (without adjuvant lipid) | 271.12 | 663.59 |
| YK-1202-LNP | 364.74 | 993.31 |
| YK-1204-LNP | 430.35 | 1938.28 |
| YK-1206-LNP | 566.69 | 1195.41 |
| YK-1208-LNP | 400.10 | 3524.32 |

### Experimental results:

As shown in Table 16 and FIG. 8, the mRNA-LNP compositions containing adjuvant lipids exhibited increased levels of cytokines IFN-γ and IL-12 in serum 6 hours after injection compared to the mRNA-LNP composition without adjuvant lipid. Specifically, the serum levels of IL-12 induced by the mRNA-LNP compositions containing YK-1202-LNP, YK-1204-LNP, YK-1206-LNP, and YK-1208-LNP were 1.3-fold, 1.6-fold, 2.1-fold, and 1.5-fold higher, respectively, than those induced by the mRNA-LNP composition without adjuvant lipid; the serum levels of IFN-γ induced by the mRNA-LNP compositions containing YK-1202-LNP, YK-1204-LNP, YK-1206-LNP, and YK-1208-LNP were 1.5-fold, 2.9-fold, 1.8-fold, and 5.3-fold higher, respectively, than those induced by the mRNA-LNP composition without adjuvant lipid.

The experimental results demonstrated that by measuring the levels of cytokines IFN-γ and IL-12 induced by the mRNA-LNP compositions containing adjuvant lipids, it can be proven that the adjuvant lipids of the present disclosure can initiate an immunostimulatory program and significantly enhance the innate immunity of the mRNA-LNP composition.

### Example 15: Therapeutic effect of mRNA-LNP compositions containing adjuvant lipids on tumor-bearing mouse model

### Experimental procedure:

1) Establishment of MC38-OVA mouse model: Female C57BL/6J mice aged 6 to 8 weeks were acclimated for at least three days prior to the study. The mice had free access to food and sterile water and were housed under a 12-hour light/dark cycle at 22°C ± 2°C and 55% ± 15% relative humidity. MC38-OVA cells were cultured in complete medium (RPMI-1640 medium supplemented with 10% fetal bovine serum (FBS)) as described in the instructions at 37°C with 5% CO₂. The cells were harvested using 0.25% trypsin-EDTA, then resuspended in Dulbecco's phosphate-buffered saline (DPBS), and transplanted into female C57BL/6J mice at 1.5 × 10⁶ cells/100 µL/mouse (MC38-OVA) by subcutaneous (SC) injection to establish a subcutaneous MC38-OVA tumor model. Vaccination was initiated when the tumor volume reached approximately 50 mm³.
2) Vaccination: The enrolled C57BL/6J mice were vaccinated with OVA mRNA-YK-1202-LNP, YK-1204-LNP, YK-1206-LNP, and YK-1208-LNP compositions containing adjuvant lipids prepared according to Example 11 via intramuscular injection on days 3, 7, 10, 12, 15, and 19 after cell injection (day 0) (each mouse received a vaccine containing 5 µg of therapeutic mRNA-OVA per injection). Mice vaccinated with an equal volume of OVA mRNA-LNP without adjuvant lipid served as the control group, with 8 mice per group in parallel.
3) Tumor size and survival rate: Starting from day 7 after tumor inoculation, tumor diameters were measured three times per week. The tumor volume of C57BL/6J mice was calculated using the following formula: V (mm³) = x × y²/2, where V represents the tumor volume, x represents the long diameter of the tumor, and y represents the short diameter of the tumor, with units in mm. Changes in body weight of C57BL/6J mice were recorded three times per week using an electronic balance, and survival rates were statistically analyzed.

### Experimental results:

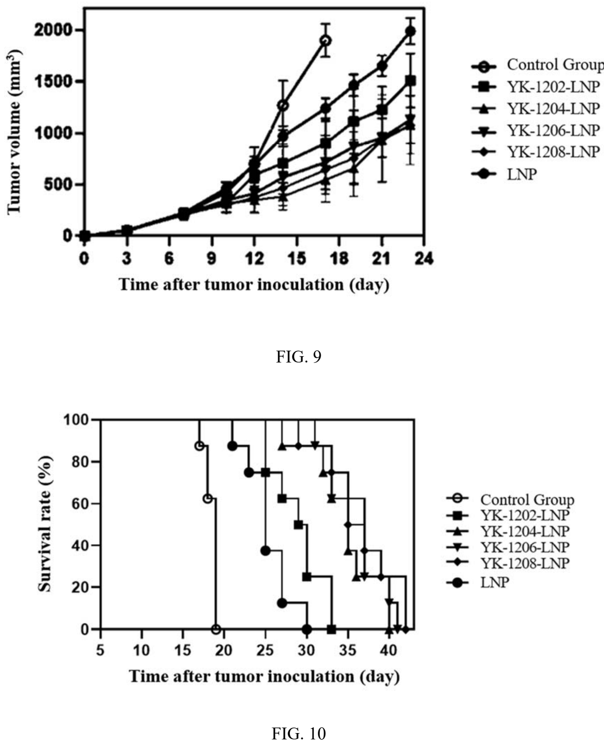

As shown in FIG. 9 and Table 17, on day 12 after tumor inoculation, mice in the blank lipid control group (Control Group) and the non-adjuvant OVA mRNA-LNP composition group entered a rapid tumor growth phase, but the gap between the non-adjuvant LNP group and the blank control group gradually widened over time. The OVA mRNA-LNP compositions with adjuvant lipids all showed significant tumor growth delay. Starting from day 14, compared to the non-adjuvant LNP group, the tumor volumes in the mRNA-LNP compositions with YK-1202, YK-1204, YK-1206, and YK-1208 also exhibited stronger tumor growth inhibition, with those with YK-1204 and YK-1208 showing relatively better tumor suppression effects.

**Table 17: Tumor growth in mice during treatment with different compositions**

| Day n | Tumor volume (mean)/mm³ | | | | | |
|---|---|---|---|---|---|---|
| | Blank lipid | LNP | YK-1202-LNP | YK-1204-LNP | YK-1206-LNP | YK-1208-LNP |
| 3 | 56.40 | 54.04 | 53.73 | 53.98 | 53.73 | 53.94 |
| 7 | 222.54 | 223.76 | 224.70 | 203.48 | 217.89 | 222.77 |
| 10 | 419.86 | 463.20 | 324.08 | 318.73 | 346.01 | 299.83 |
| 12 | 702.52 | 668.00 | 593.15 | 349.26 | 413.87 | 376.99 |
| 14 | 1269.95 | 968.57 | 707.24 | 384.34 | 569.33 | 465.62 |
| 17 | 1898.97 | 1240.78 | 899.27 | 544.98 | 716.82 | 641.81 |
| 19 | / | 1464.54 | 1113.55 | 657.29 | 866.28 | 754.28 |
| 21 | / | 1652.32 | 1228.32 | 930.67 | 955.16 | 945.47 |
| 23 | / | 1987.44 | 1509.45 | 1079.89 | 1129.42 | 1077.96 |

In terms of survival rate, as shown in FIG. 10, the mice in the blank lipid control group started to die on day 17 after tumor inoculation, with all mice dead on day 19; the mice in the non-adjuvant mRNA-LNP group started to die on day 21, with all mice dead on day 30; the mice injected with the mRNA-LNP compositions containing adjuvant lipids YK-1202, YK-1204, YK-1206, and YK-1208 started to die on days 25, 27, 31, and 29, respectively, with all mice dead on days 33, 40, 41, and 42, respectively. It can be seen that the addition of the adjuvant lipids of the present disclosure significantly prolonged the survival time of mice.

The present disclosure designed a series of novel vaccine adjuvant lipids based on Toll-like receptor 7 and 8 dual agonists, such as YK-1202, YK-1204, YK-1205, YK-1206, and YK-1208, which have at least one of the following advantages compared to the adjuvant lipids disclosed in the prior art:
1. The chemical structures of the adjuvant lipids designed in the present disclosure are all different from those of the adjuvant lipids disclosed in the prior art, representing entirely new compounds.
2. Adding the adjuvant lipids of the present disclosure to the mRNA-TLP compositions significantly enhanced the protein expression in mice while exhibiting significant spleen targeting. Compared with the adjuvant lipids in the prior art, the mRNA-TLP compositions prepared using the adjuvant lipids of the present disclosure significantly enhanced the protein expression *in vivo* and in the spleen of mice.
3. The mRNA-TLP compositions prepared using the adjuvant lipids of the present disclosure significantly increased the percentage of antigen-presenting cells (*e.g.,* B cells, pDCs, cDCs, and macrophages) expressing antigens in the spleen. Compared with the adjuvant lipids in the prior art, the mRNA-TLP compositions prepared using the adjuvant lipids of the present disclosure exhibited significantly enhanced targeting to antigen-presenting cells in the spleen of mice.
4. The mRNA-TLP compositions prepared using the adjuvant lipids of the present disclosure significantly increased the percentage of CD86-positive antigen-presenting cells in the spleen. Compared with the adjuvant lipids in the prior art, the mRNA-TLP compositions prepared using the adjuvant lipids of the present disclosure significantly enhanced the maturation status of antigen-presenting cells in the spleen of mice.
5. The mRNA-TLP compositions prepared using the adjuvant lipids of the present disclosure significantly increased the percentage of CD69-positive T cells in the spleen. Compared with the adjuvant lipids in the prior art, the mRNA-TLP compositions prepared using the adjuvant lipids of the present disclosure significantly enhanced the activation status of T cells in the spleen of mice.
6. The mRNA-TLP compositions prepared using the adjuvant lipids of the present disclosure demonstrated significant effects in controlling tumor growth and prolonging the survival time of tumor-bearing experimental animals in animal experiments compared to the mRNA-TLP composition without adjuvant lipid.
7. The mRNA-LNP compositions prepared using the adjuvant lipids of the present disclosure (2.5 to 20 mol%) exhibited favorable particle size (less than 200 nm) and uniform particle distribution (a PDI of less than 0.3). Compared with the mRNA-LNP composition without adjuvant lipid, the mRNA-LNP compositions prepared using the adjuvant lipids of the present disclosure exhibited significantly increased mRNA translation efficiency, significantly reduced cytotoxicity, and significantly enhanced protein expression in the injection site, abdominal cavity, liver, and spleen of mice, while significantly inhibiting tumor growth and prolonging the survival time of tumor-bearing experimental animals in animal experiments.

The above is a detailed description of the present disclosure, the purpose of which is to enable those skilled in the art to understand and implement the present disclosure. However, the description should not be construed as limiting the scope of protection of the present disclosure. Any equivalent changes or modifications made based on the spirit of the present disclosure shall fall within the scope of protection of the present disclosure.

## Claims

1. A compound of formula (I) or a pharmaceutically acceptable salt thereof: 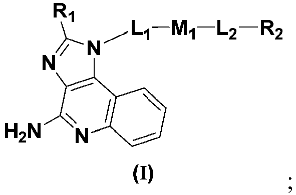
L₁ is C₁₋₁₀ alkylene or C₁₋₄ alkylene-C₆₋₁₀ aryl-C₁₋₄ alkylene;
M₁ is -NH- or -OC(O)HN-;
L₂ is C₁₋₈ alkylene, -R₃ₐC(O)OR₄ₐ-, 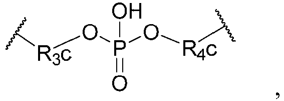 or 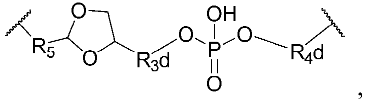 wherein R₃ₐ, R_{3c}, R_{3d}, R₄ₐ, R_{4c}, R_{4d}, and R₅ are independently C₁₋₈ alkylene;
R₁ is C₁₋₅ alkyl or C₁₋₅ alkyl substituted by C₁₋₆ alkoxy;
R₂ is 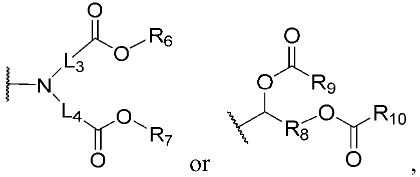
wherein L₃ and L₄ are independently C₁₋₇ alkylene;
R₆ and R₇ are independently C₇₋₂₈ alkyl;
R₈ is C₁₋₇ alkylene, and R₉ and R₁₀ are independently C₁₀₋₂₀ alkyl or C₁₀₋₂₀ alkenyl.

2. The compound of formula (I) or the pharmaceutically acceptable salt thereof according to claim 1, wherein the compound of formula (I) satisfies any one selected from the group consisting of the following conditions, or any combination of at least two thereof:
(1) L₁ is C₁₋₆ linear alkylene, and L₂ is C₁₋₆ linear alkylene-C(O)O-C₁₋₄ linear alkylene" or 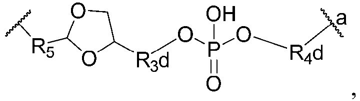 wherein the a-terminus is connected to R²; R_{3d}, R_{4d}, and R₅ are independently C₁₋₄ linear alkylene; or, L₁ is C₁₋₄ linear alkylene-C₆₋₁₀ aryl-C₁₋₄ linear alkylene, and L₂ is C₁₋₄ linear alkylene or C₁₋₆ linear alkylene-C(O)O-C₁₋₄ linear alkylene^{a}, wherein the a-terminus is connected to R²;
(2) R₁ is C₁₋₅ linear alkyl;
(3) L₃ is C₁₋₄ linear alkylene;
(4) L₄ is C₄₋₆ linear alkylene;
(5) R₆ is C₈₋₁₂ linear alkyl;
(6) R₇ is C₁₆₋₂₀ branched alkyl;
(7) R₈ is C₁₋₄ linear alkylene;
(8) R₉ and R₁₀ are independently C₁₆₋₂₀ alkenyl.

3. The compound of formula (I) or the pharmaceutically acceptable salt thereof according to claim 1, wherein the compound of formula (I) satisfies any one selected from the group consisting of the following conditions, or any combination of at least two thereof:
(1) L₁ is -(CH₂)₄-, -CH₂C((CH₃)₂)-^{b}, or 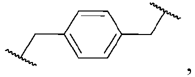 wherein the b-terminus is connected to M₁;
(2) M₁ is -NH-;
(3) L₂ is -(CH₂)₂-, -(CH₂)₃C(O)O(CH₂)₂-^{a}, -(CH₂)₅C(O)O(CH₂)₂-^{a}, 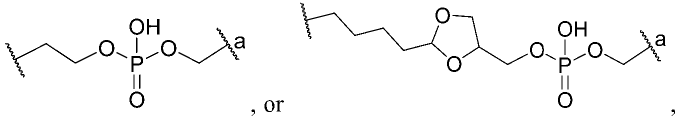 wherein the a-terminus is connected to R₂;
(4) R₁ is -(CH₂)₃CH₃ or -CH₂OCH₂CH₃;
(5) R₂ is 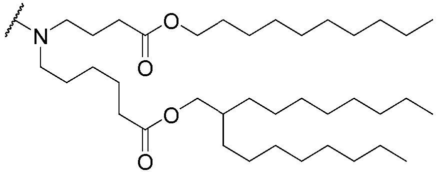 or

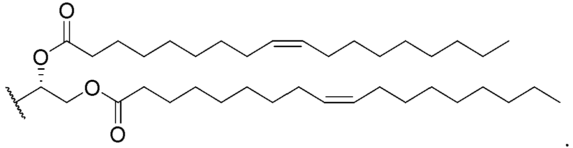

4. The compound of formula (I) or the pharmaceutically acceptable salt thereof according to claim 1, wherein L₁ is -(CH₂)₄-, and L₂ is -(CH₂)₅C(O)O(CH₂)₂-^{a} or 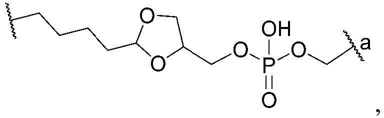 wherein the a-terminus is connected to R₂;
or, L₁ is 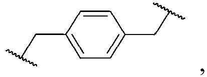 and L₂ is -(CH₂)₂-, -(CH₂)₃C(O)O(CH₂)₂-^{a}, or - (CH₂)₅C(O)O(CH₂)₂-^{a}, wherein the a-terminus is connected to R₂.

5. The compound of formula (I) or the pharmaceutically acceptable salt thereof according to claim 1, wherein the compound of formula (I) is any one of the following compounds: 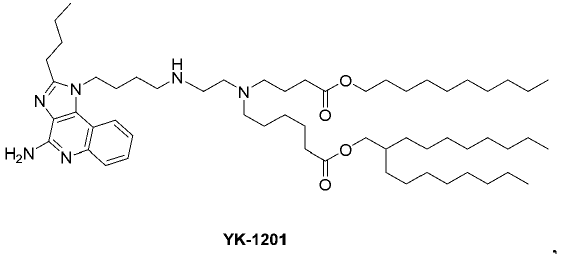 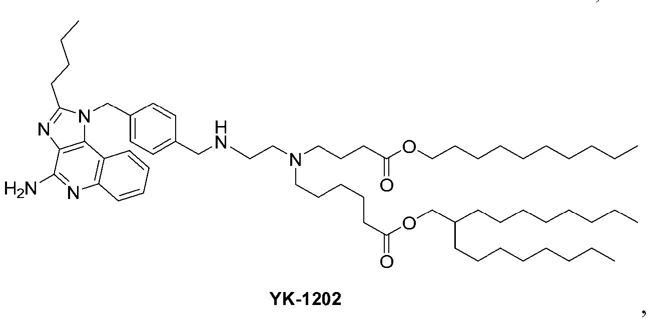 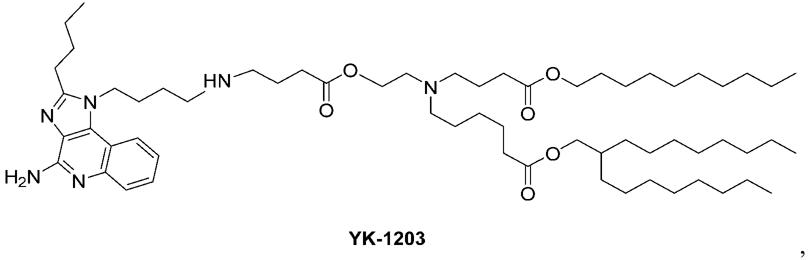 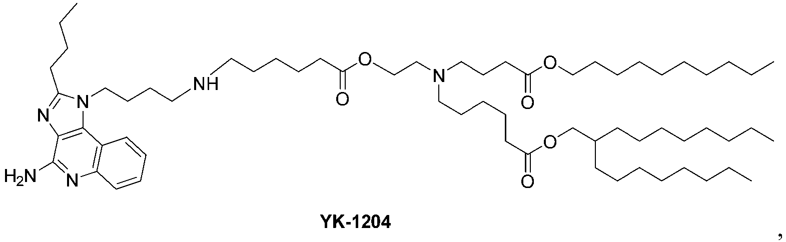 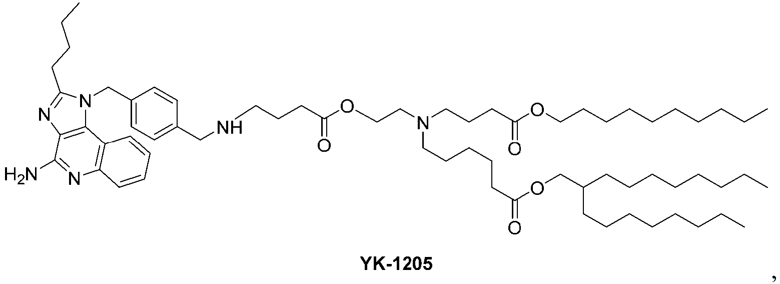 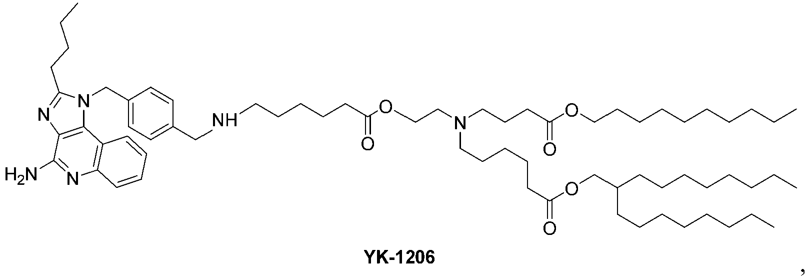 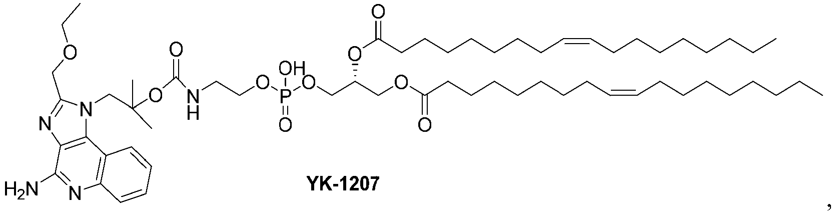 or

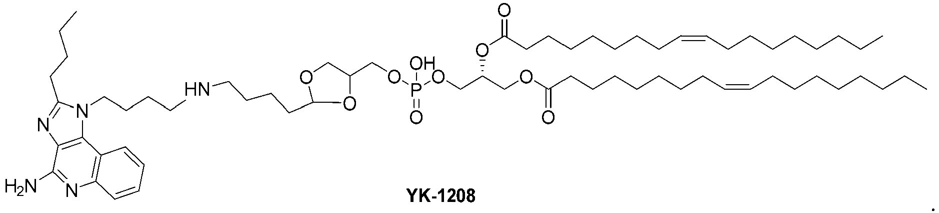

6. A lipid composition, comprising a substance Z, wherein the substance Z is the compound of formula (I) or the pharmaceutically acceptable salt thereof according to any one of claims 1 to 5.

7. The lipid composition according to claim 6, wherein the molar percentage of the substance Z in the lipid composition is 2.5 to 20%; preferably, the molar percentage of the substance Z in the lipid composition is 5%, 10%, or 15%.

8. The lipid composition according to claim 6, wherein the lipid composition comprises the substance Z, a permanent anionic lipid, a permanent cationic lipid, and a neutral lipid.

9. The lipid composition according to claim 8, wherein the lipid composition satisfies any one selected from the group consisting of the following conditions, or any combination of at least two thereof:
(1) the permanent anionic lipid is any one selected from the group consisting of 2-acetamidoethyl ((R)-2,3-bis(oleoyloxy)propyl) phosphate, (R)-2,3-bis(oleoyloxy)propyl-(2-(3-ethylthioureido)ethyl) phosphate, (R)-2,3-bis(oleoyloxy)propyl-(2-(3-ethylureido)ethyl) phosphate, (R)-2,3-bis(oleoyloxy)propyl-(2-(3-propylureido)ethyl) phosphate, (R)-2,3-bis(oleoyloxy)propyl-(2-(3-butylureido)ethyl) phosphate, and salts thereof, or any combination of at least two thereof;
(2) the permanent cationic lipid is any one selected from the group consisting of 1,2-di-O-octadecenyl-3-trimethylammonium propane, (2,3-dioleoyloxypropyl)trimethylammonium, and salts thereof, or any combination of at least two thereof;
(3) the neutral lipid is any one selected from the group consisting of 1,2-dioleoyl-sn-glycero-3-phosphoethanolamine, 1,2-distearoyl-sn-glycero-3-phosphocholine, and salts thereof, or any combination of at least two thereof;
(4) the molar percentage ratio of the substance Z, the permanent anionic lipid, the permanent cationic lipid, and the neutral lipid is (2.5 to 20):(10 to 33):(20 to 60):(20 to 40).

10. The lipid composition according to claim 8, wherein the lipid composition satisfies any one selected from the group consisting of the following conditions, or any combination of at least two thereof:
(1) the permanent anionic lipid is sodium 2-acetamidoethyl ((R)-2,3-bis(oleoyloxy)propyl) phosphate;
(2) the molar percentage of the permanent anionic lipid in the lipid composition is 25%;
(3) the permanent cationic lipid is 1,2-di-O-octadecenyl-3-trimethylammonium propane chloride salt;
(4) the molar percentage of the permanent cationic lipid in the lipid composition is 30%, 35%, 40%, 45%, or 47.5%;
(5) the neutral lipid is 1,2-dioleoyl-sn-glycero-3-phosphoethanolamine;
(6) the molar percentage of the neutral lipid in the lipid composition is 25%.

11. The lipid composition according to claim 6, wherein the lipid composition comprises the substance Z, a cationic lipid, a neutral lipid, a structured lipid, and a polymer-conjugated lipid.

12. The lipid composition according to claim 11, wherein the molar percentage ratio of the substance Z, the cationic lipid, the neutral lipid, the structured lipid, and the polymer-conjugated lipid is (2.5 to 20):(25 to 75):(5 to 25):(15 to 65):(0.5 to 10);
or, the cationic lipid is any one selected from the group consisting of the following compounds in (1) to (7), or any combination of at least two thereof:
(1) a compound of formula (II) or a pharmaceutically acceptable salt thereof, wherein G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl; 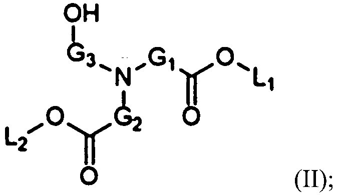
(2) a compound of formula (III) or a pharmaceutically acceptable salt thereof, wherein G₁ is C₂₋₈ alkylene; G₂ is C₂₋₈ alkylene; L₁ is -C(O)O- or -OC(O)-; L₂ is -C(O)O- or -OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or -(CH₂)₄-; 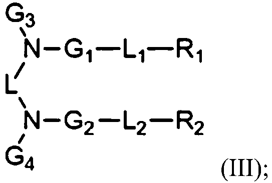
(3) a compound of formula (IV) or a pharmaceutically acceptable salt thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-; 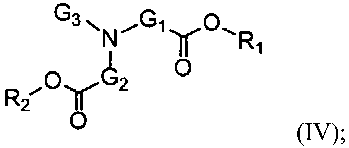
(4) a compound of formula (V) or a pharmaceutically acceptable salt thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is -CH₃, -CH₂CH₃, or - CH₂CH₂OH; 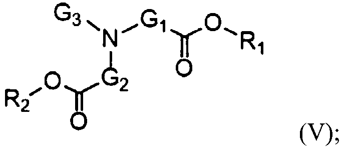
(5) a compound of formula (VI) or a pharmaceutically acceptable salt thereof, wherein G¹ and G² are each independently C₆₋₁₀ alkylene; G₃ is C₁₋₁₂ alkylene; R¹ and R² are each independently C₆₋₂₄ alkyl or C₆₋₂₄ alkenyl; R³ is OR⁵, N, -C(=O)OR⁴, -OC(=O)R⁴, or - NR⁵C(=O)R⁴; R⁴ is C₁₋₁₂ hydrocarbyl; R⁵ is H or C₁₋₆ hydrocarbyl; 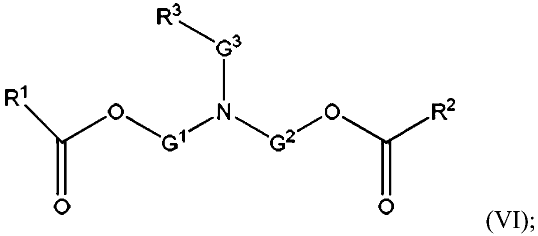
(6) a compound of formula (VII) or a pharmaceutically acceptable salt thereof, wherein R₄ is -(CH₂)_{nQ} or -(CH₂)ₙCHQR; Q is -OR, -OH, -O(CH₂)ₙN(R)₂, -OC(O)R, -CX₃, -CN, - N(R)C(O)R, -N(H)C(O)R, -N(R)S(O)₂R, -N(H)S(O)₂R, -N(R)C(O)N(R)₂, -N(H)C(O)N(R)₂, - N(H)C(O)N(H)(R), -N(R)C(S)N(R)₂, -N(H)C(S)N(R)₂, -N(H)C(S)N(H)(R), -N(R)S(O)₂R, or heterocycle; n is 1, 2, or 3; R is C₁₋₈ alkyl; X is H or C₁₋₈ alkyl; 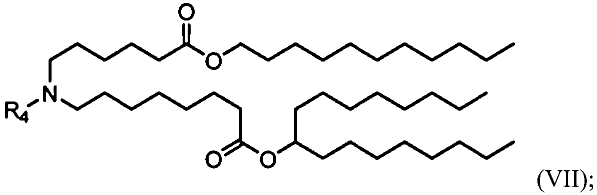
(7) a compound of formula (VIII) or a pharmaceutically acceptable salt thereof, 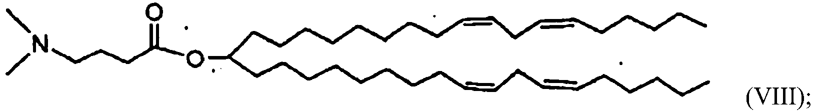
or the neutral lipid is any one selected from the group consisting of phosphatidylcholine, phosphatidylethanolamine, sphingomyelin, ceramide, and sterol, or any combination of at least two thereof.

13. The lipid composition according to claim 11, wherein the lipid composition satisfies any one selected from the group consisting of the following conditions, or any combination of at least two thereof:
(1) the cationic lipid is any one selected from the group consisting of YK-009, YK-401, YK-305, ALC0315, SM102, and DLIN-MC3-DMA, or any combination of at least two thereof: 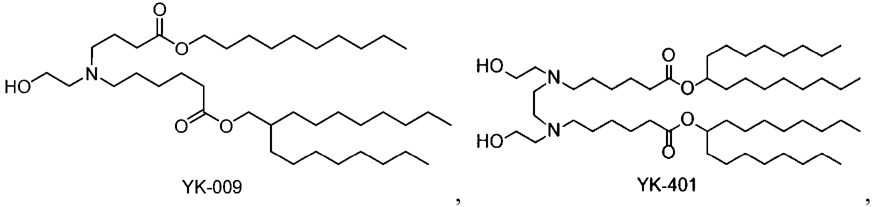 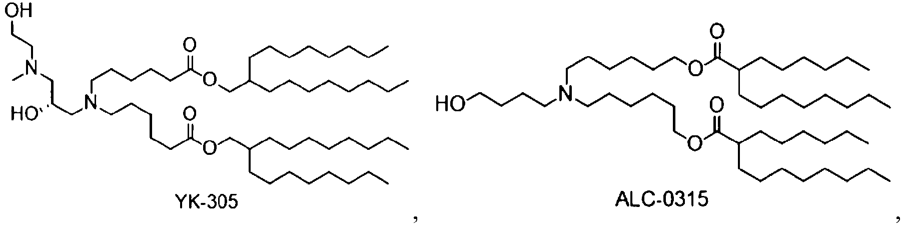 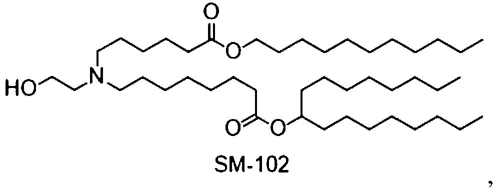 and 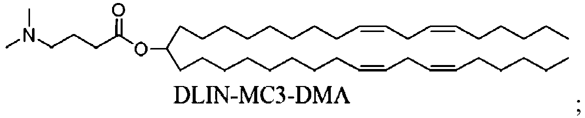
(2) the neutral lipid is any one selected from the group consisting of 1,2-dilinoleoyl-sn-glycero-3-phosphocholine, 1,2-dimyristoyl-sn-glycero-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt, dipalmitoyl phosphatidylglycerol, palmitoyl oleoyl phosphatidylethanolamine, distearoyl-phosphatidyl-ethanolamine, dipalmitoyl phosphatidylethanolamine, dimyristoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, and lysophosphatidylethanolamine, or any combination of at least two thereof;
(3) the structured lipid is any one selected from the group consisting of cholesterol, nonsterol, sitosterol, ergosterol, campesterol, stigmasterol, brassicasterol, tomatine, ursolic acid, α-tocopherol, and corticosteroid, or any combination of at least two thereof;
(4) the polymer-conjugated lipid is any one selected from the group consisting of distearoyl phosphatidylethanolamine polyethylene glycol 2000, 1,2-dimyristoyl-sn-glycero-3-methoxypolyethylene glycol 2000, and methoxypolyethylene glycol ditetradecylacetamide, or any combination of at least two thereof;
preferably, the lipid composition satisfies any one selected from the group consisting of the following conditions, or any combination of at least two thereof:
(1) the cationic lipid is YK-009;
(2) the molar percentage of the cationic lipid in the lipid composition is 30%, 35%, 40%, 45%, or 47.5%;
(3) the neutral lipid is 1,2-dioleoyl-sn-glycero-3-phosphoethanolamine and/or 1,2-distearoyl-sn-glycero-3-phosphocholine;
(4) the molar percentage of the neutral lipid in the lipid composition is 10%;
(5) the structured lipid is cholesterol;
(6) the molar percentage of the structured lipid in the lipid composition is 38.5%;
(7) the polymer-conjugated lipid is 1,2-dimyristoyl-sn-glycero-3-methoxypolyethylene glycol 2000;
(8) the molar percentage of the polymer-conjugated lipid in the lipid composition is 1.5%.

14. A pharmaceutical composition, comprising:
(A) a therapeutic and/or prophylactic agent, comprising any one of the group consisting of a nucleic acid molecule, a small molecule compound, a polypeptide, or a protein, or any combination of at least two thereof;
(B) the lipid composition according to any one of claims 8 to 13.

15. The pharmaceutical composition according to claim 14, wherein the pharmaceutical composition satisfies any one selected from the group consisting of the following conditions, or any combination of at least two thereof:
(1) the therapeutic and/or prophylactic agent and the lipid composition according to any one of claims 8 to 10 are used in an amount such that the charge ratio of positive to negative charges in the pharmaceutical composition is 1:(2 to 5);
(2) the mass ratio of the lipid composition according to any one of claims 11 to 13 to the therapeutic or prophylactic agent is (12.5 to 25):1;
(3) the pharmaceutical composition is used for delivering the therapeutic and/or prophylactic agent to antigen-presenting cells in a target organ or tissue.
